(19) Europäisches Patentamt
European Patent Office
Office européen des brevets

(11) Publication number: **0 160 996**
**B1**

(12) # EUROPEAN PATENT SPECIFICATION

(45) Date of publication of patent specification: **23.11.88**

(51) Int. Cl.[4]: **G 03 C 5/54**, G 03 C 1/02, G 03 C 1/42, C 07 C 147/06

(21) Application number: **85105700.0**

(22) Date of filing: **09.05.85**

(54) **Heat developable light-sensitive material.**

(30) Priority: **09.05.84 JP 92558/84**

(43) Date of publication of application:
**13.11.85 Bulletin 85/46**

(45) Publication of the grant of the patent:
**23.11.88 Bulletin 88/47**

(84) Designated Contracting States:
**DE GB NL**

(56) References cited:
**EP-A-0 120 403**
**US-A-4 514 493**

**RESEARCH DISCLOSURE, no. 151, November 1976, pages 9-11, disclosure no. 15109, Havant, Hampshire, GB; "Sulfonylacetate activator-stabilizer precursor"**

(73) Proprietor: **FUJI PHOTO FILM CO., LTD.**
**210 Nakanuma Minami Ashigara-shi**
**Kanagawa 250-01 (JP)**

(72) Inventor: **Yabuki, Yoshiharu c/o Fuji Photo Film Co., Ltd.**
**No. 210, Nakanuma**
**Minami Ashigara-shi Kanagawa (JP)**
Inventor: **Kawata, Ken c/o Fuji Photo Film Co., Ltd.**
**No. 210, Nakanuma**
**Minami Ashigara-shi Kanagawa (JP)**
Inventor: **Hirai, Hiroyuki c/oFuji Photo Film Co., Ltd.**
**No. 210, Nakanuma**
**Minami Ashigara-shi Kanagawa (JP)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

**EP 0 160 996 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a heat developable light-sensitive material containing a base precursor.

A heat developable light-sensitive material usually contains a base or a base precursor in the light-sensitive material for accelerating the development by heating. Also, in this case, it is preferred to use a base precursor capable of releasing a basic material by heat decomposition from the viewpoint of the shelf life of the light-sensitive material.

Typical examples of the base precursor are described in GB—A—998,949. Preferred base precursors are salts of carboxylic acids and organic bases. Useful carboxylic acids inlcude trichloroacetic acid, trifluoroacetic acid, etc., and useful organic bases include guanidine, piperidine, morpholine, p-toluidine, 2-picoline, etc. Guanidine trichloroacetate described in US—A—3,220,846 is particularly useful for such a purpose. Also, the aldonamides described in Japanese Patent Application (OPI) No. 22,625/'75 are decomposed at high temperature to form a base and are preferably used (the term "OPI" indicates an unexamined published patent application open to public inspection).

However, many of these base precursors require a relatively long period of time for obtaining images and cause severe fog. Also, these base precursors have the disadvantages that they are easily influenced by air and humidity, to decompose and to change photographic characteristics of the light-sensitive material, or greatly reduce the shelf life of the light-sensitive materials containing them.

For overcoming such a disadvantage, sulfonylacetates are proposed in Japanese Patent Application (OPI) No. 168,441/'84. These materials are excellent from the viewpoint of providing images having a high density in a short period of time, but are still not totally sufficient in providing high density, or cause a side action of forming severe fog. Furthermore, some of the above-described base precursors are insufficient with respect to the shelf life of the light-sensitive material before heat development.

EP—A—120403 discloses a heat developable color photographic material comprising a support having thereon at least a photosensitive silver halide, a binder, a dye-releasing material which is reductive to the photosensitive silver halide and releases a hydrophilic dye upon reaction with the photosensitive silver halide by heating, and a base precursor.

It is the object of this invention to provide a heat developable light-sensitive material containing a novel base precursor capable of giving images having high density and less fog, said light-sensitive material being excellent in stability with the passage of time, and a method for producing an image.

The terminology "stability with the passage of time" refers to the time during storage of a heat developable light-sensitive material before heat development, the heat developable light-sensitive material shows less change of photographic performance such as the maximum density, the minimum density and sensitivity.

Said object is attained by a heat developable light-sensitive material comprising a support and formed thereon a silver halide emulsion layer, wherein said light-sensitive material contains at least one compound selected from the group consisting of compounds represented by formula (I) or (II) as a base precursor:

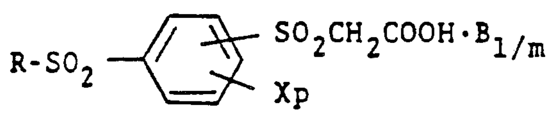
(I)

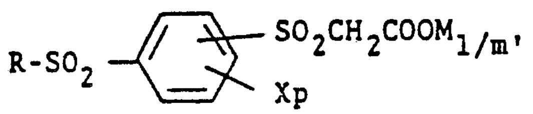
(II)

wherein R represents a substituted or unsubstituted alkyl group, a substituted or unsubstituted cycloalkyl group, a substituted or unsubstituted alkenyl group, a substituted or unsubstituted alkynyl group, a substituted or unsubstituted aralkyl group, a substituted or unsubstituted aryl group or a heterocyclic group provided that R is not carboxymethyl; X represents a group or atom selected from the group consisting of a hydrogen atom, a halogen atom, an alkyl group, a cycloalkyl group, an aralkyl group, an alkoxy group, an aryloxy group, an aryl group, an alkyl or aryl acylamino group, an alkyl or aryl acyl group, a cyano group, an alkylsulfonylamino group, a nitro group, an arylsulfonylamino group, an alkylsulfonyl group, an arylsulfonyl group, a sulfamoyl group, a substituted sulfamoyl group, a carbamoyl group, a substituted carbamoyl group, an alkylthio group, an arylthio group, a alkoxycarbonyl group, an aryloxycarbonyl group, an alkyl or arylacyloxy group, and substituted groups thereof at the alkyl or aryl moiety thereof, $—OM_{l/m'}$, $—COOM_{l/m'}$, $—OH_{l/m}$ (wherein —OH is phenolic) and $—COOH{\cdot}B_{l/m}$, and a method of producing an image, which comprises heat developing said heat developable light-sensitive material.

In formulae (I) and (II), the alkyl group, cycloalkyl group, alkenyl group, and alkynyl group represented by R preferably have from 1 to 8 carbon atoms, and the aralkyl group and aryl group represented by R preferably have at most 8 carbon atoms. It is preferred that the heterocyclic ring shown by R has at least one of nitrogen atom, an oxygen atom and a sulfur atom as a hetero atom of a 5- or 6-membered heterocyclic ring. Each group represented by R may be substituted and preferred examples of the

substituent are an alkyl group, an alkyl or aryl sulfonyl group, a sulfamoyl group, an N-alkyl- or N-arylsulfamoyl group, a carbamoyl group, an N-alkyl or an arylcarbamoyl group, an alkyl or arylsulfonamide group, an alkyl or arylacylamino group, a halogen atom, and a hydroxycarbonyl group. These substituents may further have a substituent selected from groups disclosed hereinafter for X.

Specific examples of the groups shown by R are a methyl group, an ethyl group, a butyl group, an octyl group, a 2-ethylhexyl group, a phenyl group, a p-chlorophenyl group, a p-bromophenyl group, a p-methylphenyl group, a m-(diethylsulfamoyl)phenyl group, a 1-naphthyl group, a 2-naphthyl group, a benzyl group, a pyridyl group, a pyrazolyl group and an imidazolyl group.

It is desirable that B is a base which is conventionally used in a heat developable light-sensitive material, that is, a mono- or di-acidic base having a pKa of 7 or higher and having not more than 12 carbon atoms, and B is preferably a low volatile base having a pKa of 10 or higher and a boiling point of 150°C or higher. More preferably, B represents organic bases, especially, a monoacidic or diacidic nitrogen-containing sulfur-free base, for example, aromatic or aliphatic amines or diamines, piperidines piperazines, guanidines, cyclic guanidines, amidines, cyclic amidines or tetraalkylammonium hydroxides. Preferred examples of B are dimethylamine, diethylamine, piperidine, piperadine, ethylenediamine, N,N'-dimethylethylenediamine, acetamidine, diazabicyclononene, diazabicycloundecene, tetramethylammonium hydroxide, tetraethylammonium hydroxide,

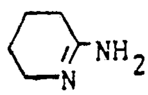, 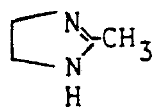, 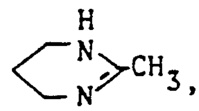, 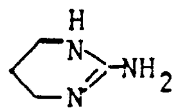

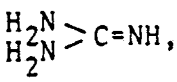, 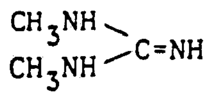, 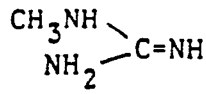, 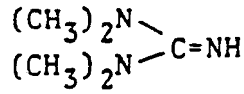,

N,N,N',N'-tetramethylethylenediamine or N,N,N',N'-tetramethyltetramethylenediamine.

If X presents an alkyl group and an aryl group said groups may be further substituted.

Preferred examples of M are Na+, K+, Cs+ and Ba++.

It is preferred that the carboxymethylsulfonyl group be at a para-position to the position of the sulfonyl group ($RSO_2$—).

Specific examples of the basic precursor are illustrated below.

1)
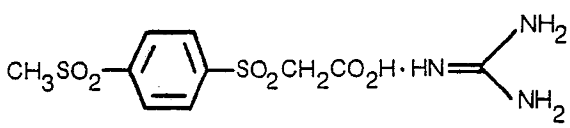

2)
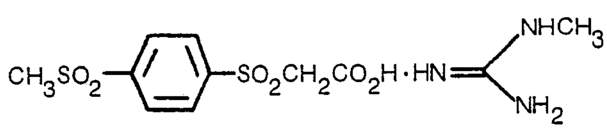

3)
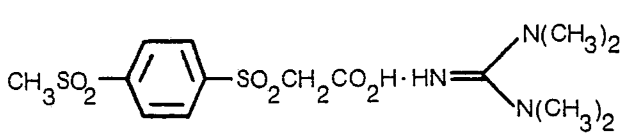

4)
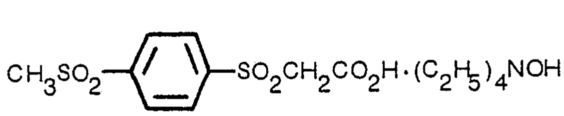

5)
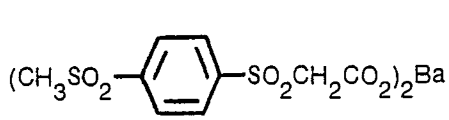

6)
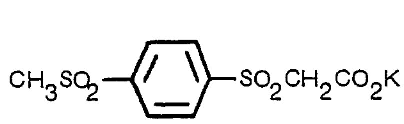

7)

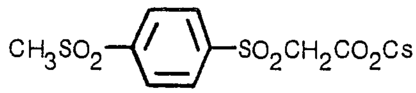

8)

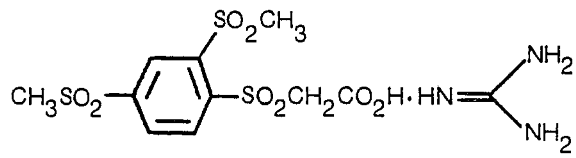

9)

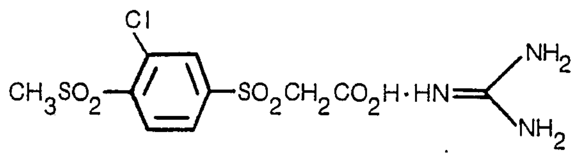

10)

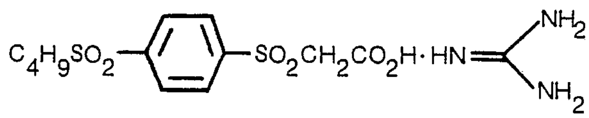

11)

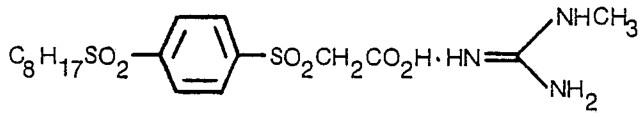

12)

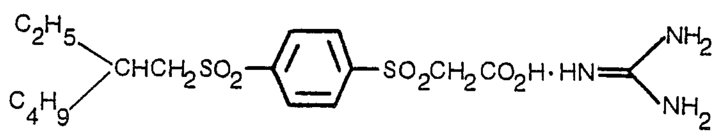

13)

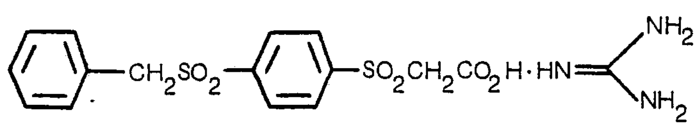

14)

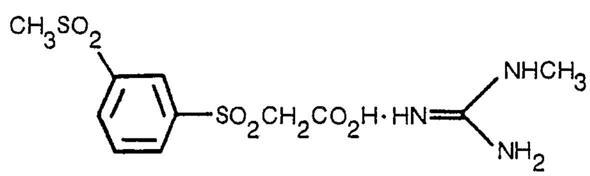

15)

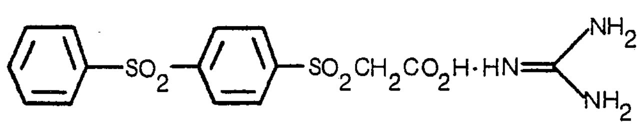

16)

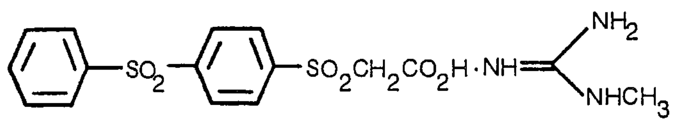

17)

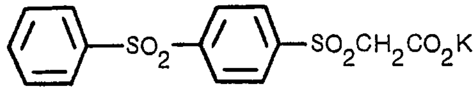

18)

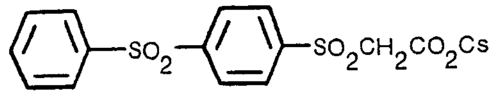

19)

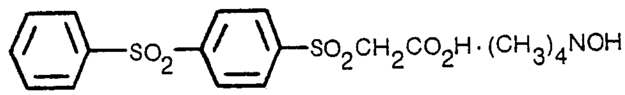

20)

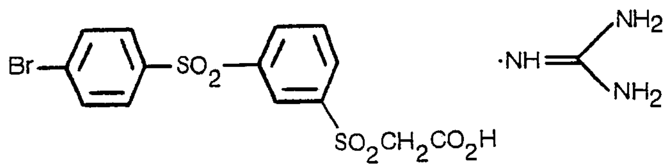

21)

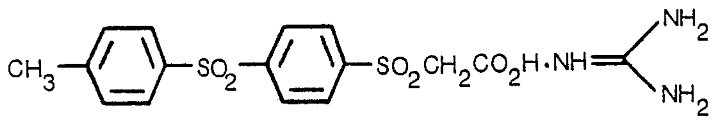

22)

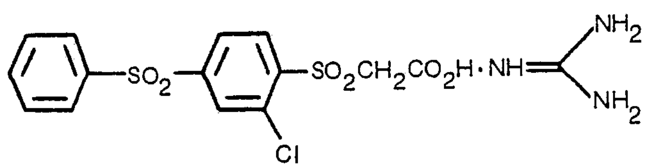

23)

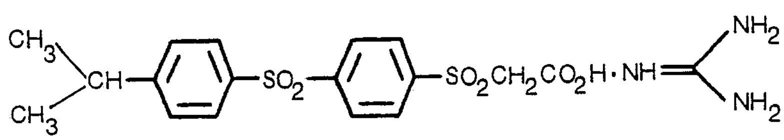

24)

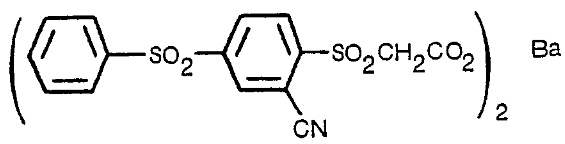

25)

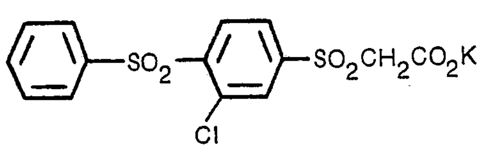

26)

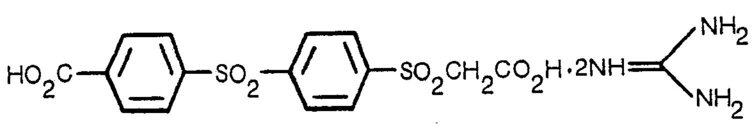

27)

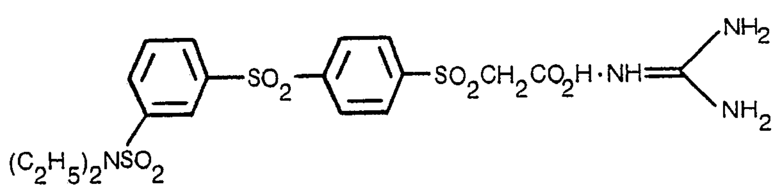

28)

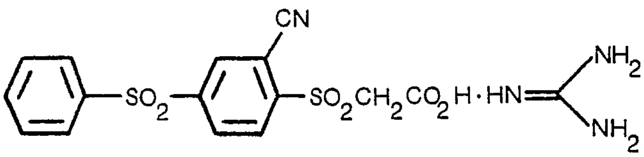

The base precursors of this invention can be prepared by the following synthesis methods.

(a) An arylsulfone having an appropriate releasable group (e.g., halogen) is reacted with thioglycolic acid in the presence of a base to form a sulfide and then the sulfide is oxidized to form α-sulfonylacetic acid. The salt forming method is conventional.

b) An arylsulfone is reacted with chlorosulfonic acid and the reaction product is reduced with sodium sulfite to form sulfinic acid, which is condensed with an α-haloacetic acid ester to form an α-sulfonyl acetic acid ester. Then, by alkali-hydrolyzing the product at room temperature, α-sulfonylacetic acid is obtained. The salt formation is performed by a conventional method.

Then, practical synthesis examples are shown below.

Synthesis Example 1

Synthesis of Base Precursor (1):

To 600 ml of chlorobenzene were added 272 g of anhydrous aluminum chloride and 220 g of methanesulfonyl chloride and the mixture was heated on a steam bath for 6 hours with stirring. The solution thus formed was poured into ice-water, and then the product was extracted with 400 ml of methylene chloride. Methylene chloride was distilled off from the extract under reduced pressure. To the residue thus formed was added 300 ml of methanol, the mixture was cooled to 15°C, and crystals thus deposited were collected by filtration to provide 212 g 1-chloro-4-methylsulfonyl benzene having a melting point of 92 to 94°C.

To 1.6 liters of dimethylformamide were added 144 g of thioglycolic acid (85% in purity) and 532 g of a 28% methanol solution of sodium methoxide to form the disodium salt of thioglycolic acid. To the salt was added 200 g of 1-chloro-4-methylsulfonyl benzene prepared above and while distilling off methanol, the mixture was heated to 100°C for 6 hours with stirring, whereby a large amount of a white salt deposited. The reaction mixture thus formed was added to an aqueous hydrochloric acid solution and the product thus formed was extracted twice, each time with 400 ml of methylene chloride.

Then, the methylene chloride was distilled off under reduced pressure, and to the residue thus formed were added 270 ml of toluene and 30 ml of ethyl acetate. The reaction mixture was cooled to 5°C and p-methylsulfonylphenylsulfonylacetic acid thus deposited was collected by filtration. The amount of the product was 120 g and the melting point was 136 to 139°C.

To 600 ml of water were added 120 g of p-methylsulfonylphenylsulfonylacetic acid and 1 g of sodium tungstinate, and then 120 g of 35% hydrogen peroxide was added dropwise thereto at a temperature below 85°C. Furthermore, after stirring the mixture, the reaction mixture was cooled and crystals thus deposited were collected by filtration to provide 130 g of p-methylsulfonylphenylsulfonylacetic acid. The melting point thereof was 191 to 193°C (decompd.).

To a mixture of 27.8 g of p-methylsulfonylphenylsulfonylacetic acid and 140 ml of methanol was added dropwise with care an aqueous solution of about 9 g of guanidine carbonate to neutralize the compound. Crystals thus formed were collected to provide 29.0 g of Base Precursor (1). The melting point was 246 to 250°C (decompd.).

Synthesis Example 2

Synthesis of Base Precursor (20)

From 200 g of anhydrous aluminum chloride, 153 ml of benzenesulfonyl chloride, 138 ml of bromobenzene, and 600 ml of methylene chloride was prepared 220 g of p-bromophenyl-phenyl-sulfone according to the method of Journal of Chem. Soc., p 2508 (1960). To 100 ml of chlorosulfonic acid was added 100 g of p-bromophenylphenylsulfone, and after stirring the mixture for 4 hours at 130 to 140°C, the mixture was poured into ice water to provide 121 g of 3-(4-bromophenylsulfonyl)benzenesulfonyl chloride.

To 150 ml of water was added 85 g of sodium sulfite and then 54 g of 3-(4-bromophenylsulfonyl)-benzenesulfonyl chloride was added thereto. After stirring the mixture for 2 hours at 50°C, the reaction mixture thus obtained was ice-cooled and crystals thus formed were collected by filtration. To the crystals thus obtained were added 40 ml of water, 40 ml of acetonitrile and 15 ml of methyl chloroacetate followed by refluxing for 2 hours. The reaction mixture thus formed was extracted with chloroform and then chloroform was distilled off from the extract to provide 32.7 g of oily 3-(4-bromophenylsulfonyl)phenylsulfonylacetic acid methyl ester. Then, the oily product thus obtained was added to a mixture of 60 ml of water, 60 ml of methanol, and 8.1 g of potassium hydroxide and the resultant mixture was stirred for one hour at room temperature. After adding 20 ml of 35% hydrochloric acid to the reaction mixture, the mixture was ice-cooled and crystals thus deposited were collected by filtration to provide 25.4 g of 3-(4-bromophenylsulfonyl)phenylsulfonylacetic acid. By neutralizing 21 g of the crystals thus obtained with 4.5 g of guanidine carbonate as in Synthesis Example 1, 22.3 g of Base Precursor (20) was obtained. The melting point was 85°C and the decomposition point was 95°C.

Other base precursors could be also prepared in an analogous manner as above, and the melting points of typical precursors are shown in the following table.

| Base Precursor | Melting Point |
|---|---|
| (1) | 246—250°C (decomd.) |
| (2) | 232—236°C (decomd.) |
| (6) | above 250°C |
| (8) | 190°C (decomd.) |
| (9) | 106°C (decomd.) |
| (11) | 172—175°C (decomd.) |
| (12) | 91°C (decomd.) |
| (13) | above 250°C |
| (15) | 137—139°C (decomd.) |
| (16) | 132—133°C (decomd.) |
| (20) | 85°C (decomd. at 95°C) |
| (21) | 168—170°C (decomd.) |
| (23) | 175—178°C |
| (26) | 85—90°C (decomd.) |
| (27) | 125—128°C (decomd. at 170°C) |
| (28) | 175—180°C |

In the present invention a heat developable silver halide light-sensitive material is used as a heat developable light-sensitive material. The silver halide light-sensitive material comprises a support having coated thereon at least one silver halide emulsion layer comprising a silver halide dispersed in a binder. The silver halide light-sensitive material may further have at least one of an interlayer, a filter layer, an antihalation layer, a protective layer, and an image fixing (receiving) layer.

The base precursor of the present invention may be incorporated into any of these layers; however, it is preferably incorporated in a silver halide emulsion layer. When the fixing layer is provided on a different support from that on which the silver halide emulsion layer is coated, the base precursor may be incorporated to this fixing layer. In this case a fixing material having a fixing layer and a heat developable light-sensitive material are used in combination.

Examples of the silver halide include silver chloride, silver chlorobromide, silver chloroiodide, silver bromide, silver iodobromide, silver chloroiodobromide, silver iodide. An organic silver salt such as silver benzotriazole may also be incorporated to a silver halide emulsion as an oxidizing agent.

The base precursor is incorporated into at least one of the above described layers by dissolving it into water or an organic solvent having a low boiling point (such as methanol) or a high boiling point (such as those disclosed hereinafter as a solvent for a dye providing material), and dissolving or dispersing the thus obtained solution to a coating composition for the layer. The base precursor may also be dispersed in a binder solution directly.

The effect of the base precursor of this invention is particularly remarkable when used together with a light-sensitive silver halide emulsion that has been spectrally sensitized. That is, when the precursor is used together with a spectrally sensitized light-sensitive silver halide emulsion, the extent of increasing the image density is particularly high.

The spectral sensitization of silver halide emulsions is performed using methine dyes, etc. Examples of dyes useful for spectral sensitization include cyanine dyes, merocyanine dyes, complex cyanine dyes, complex merocyanine dyes, holopolar cyanine dyes, hemicyanine dyes, styryl dyes, and hemioxonole dyes. Particularly useful dyes are cyanine dyes, merocyanine dyes, and complex merocyanine dyes. For

these dyes, nuclei usually utilized for cyanine dyes can be applied as basic heterocyclic ring nuclei. For example, there are pyrroline nuclei, oxazoline nuclei, thiazoline nuclei, pyrrole nuclei, oxazole nuclei, thiazole nuclei, selenazole nuclei, imidazole nuclei, tetrazole nuclei and pyridine nuclei; the nuclei formed by the fusion of aliphatic hydrocarbon rings to the foregoing nuclei and the nuclei formed by the fusion of aromatic hydrocarbon rings to the foregoing nuclei, such as indolenine nuclei, benzindolenine nuclei, indole nuclei, benzoxazole nuclei, naphthoxazole nuclei, benzothiazole nuclei, naphthothiazole nuclei, benzoselenazole nuclei, benzimidazole nuclei and quinoline nuclei. These nuclei may be substituted on carbon atoms.

For the merocyanine dye or complex merocyanine dye can be applied a 5- or 6-membered heterocyclic nucleus such as a pyrazolin-5-one nucleus, thiohydantoin nucleus, a 2-thioaxazolidine-2,4-dione nucleus, a thiazolidine-2,4-dione nucleus, a rhodanine nucleus or a thiobarbituric acid nucleus, as a nucleus having a ketomethylene structure.

These sensitizing dyes may be used individually or as a combination thereof. A combination of sensitizing dyes if frequently used for supersensitization.

Examples of useful sensitizing dyes are described in, for example, DE—A—929,080; US—A—2,493,748; 2,503,776; 2,519,001; 2,912,329; 3,656,959; 3,672,897; 3,694,217; 4,025,349; 4,046,572; GB—A—1,242,588; Japanese Patent Publication Nos. 14,030/'69 and 24,844/'77.

The amount of the sensitizing dye is from 0.001 g to 20 g, and preferably from 0.01 g to 2 g per 100 g of silver of the silver halide emulsion.

The base precursor of this invention can be used in an amount of a wide range. The useful range is not more than 50% by weight, usually not less than 0.001% by weight, preferably 0.01% by weight to 40% by weight of the total weight of the dry coated layer of a light-sensitive material.

In this invention, silver may be used as the image-forming material or various image-forming materials may be used in various manners.

For example, couplers which form color images by combining with the oxidation product of a developing agent which is conventionally used for liquid development can be used as the image-forming materials in this invention. This includes 5-pyrazolone couplers, pyrazolobenzimidazole couplers, cyano-acetylcumarone couplers and open chain acylacetonitrile couplers, as magenta couplers; acylacetamido couplers (e.g., benzoylacetanilides or pyvaloylacetanilides), as yellow couplers; and naphthol couplers or phenol couplers, as cyan couplers. Non-diffusible couplers having a hydrophobic group, a so-called ballast group, in the molecule, or polymerized couplers are preferred. The couplers may be four equivalent or two equivalent to silver. Also, the couplers may be colored couplers having a color correction effect or so-called DIR couplers capable of releasing a development inhibitor with the progress of development.

Also, dyes forming positive color images by a photographic silver dye bleaching process, for example, the dyes described in Research Disclosure, April 1976, pages 30—32 (RD—14433), ibid., December 1976, pages 14—15 (RD—15227), US—A—4,235,957 and the leuco dyes described in US—A—3,985,565; 4,022,617.

Also, the dyes having introduced therein a nitrogen-containing heterocyclic ring group described in Reserach Disclosure, May 1978, pages 54—58, (RD—16966) can be used.

Furthermore, a dye-providing material releasing a mobile dye by utilizing a coupling reaction with a reducing agent oxidized by an oxidation reduction reaction with silver halide or an organic silver salt upon heat development at high temperature described in EP—A—79,056 and 67,455; DE—A—3,217,853, etc., and a dye-providing material releasing a mobile dye as the result of an oxidation-reduction reaction with silver halide or an organic silver salt upon heat development at a high temperature described in EP—A—76,492, 66,282, 120,306 and 119,470; DE—A—3,215,485, can be used.

The dye providing materials used in this invention are preferably shown by following formula (CI)

$$(Dye\text{-}X)_q\text{—}Y \qquad (CI)$$

wherein Dye represents a dye moiety which becomes a mobile dye when released from the molecule and has, preferably, a hydrophilic group. As such dyes, there are an azo dye, an azomethine dye, an anthraquinone dye, a naphthoquinone dye, a styryl dye, a nitro dye, a quinoline dye, a carbonyl dye or a phthalocyanine dye, and specific examples of them are shown below. In addition, these dyes can be used in the form of having their absorption temporarily shifted to a short wavelength side, which can recover the original color by development.

For example, the dyes described in EP—A—76,492 can be thus utilized.

X in the aforesaid formula represents a simple bond or a linkage group such as, for example, a —NR— group (wherein R represents a hydrogen atom, an alkyl group or a substituted alkyl group), a —$SO_2$— group, a —CO— group, an alkylene group, a substituted alkylene group, a phenylene group, a substituted phenylene group, a naphthylene group, a substituted naphthylene group, an —O— group a —SO— group, or a group formed by the combination of two or more of the above-described groups.

Y in the above formula represents a group having a property of releasing Dye in direct or inverse proportion to a light-sensitive silver salt having imagewise latent images to form a difference in diffussibility between the released dye and the compound shown by Dye-X—Y.

The group represented by Y is now explained in detail.

Y in formula (CI) described above is selected so that the compound represented by formula (CI) becomes a non-diffusible image-forming compound which is oxidized and self-cleaved as result of the development to provide a diffusible dye.

An example of Y effective for this type of compound is an N-substituted sulfamoyl group. An example of Y is a group represented by formula (CII)

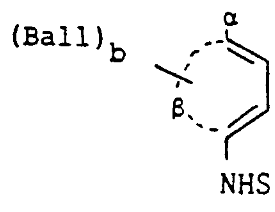

(CII)

wherein β represents a nonmetallic atomic group necessary for forming a benzene ring, said benzene ring may be condensed with a carbon ring or a heterocyclic ring to form, for example, a naphthalene ring, a quinoline ring, a 5,6,7,8-tetrahydronaphthalene ring or a chroman ring.

α in formula (CII) represents a group represented by —$OG^{11}$ or —$NHG^{12}$ (wherein $G^{11}$ represents a hydrogen atom or a group forming a hydroxy group by hydrolysis) and $G^{12}$ represents a hydrogen atom, an alkyl group having from 1 to 22 carbon atoms, or a hydrolyzable group, and Ball represents a ballast group.

Practical examples of Y are described in Japanese Patent Application (OPI) Nos. 33,826/'73 and 50,736/'78.

Another example of Y suitable for the compound of this type is the group represented by formula (CIII)

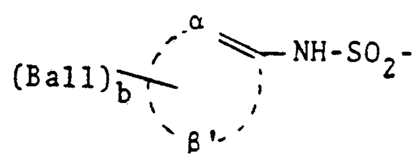

(CIII)

wherein Ball, α, and b have the same meaning as in the case of formula (CII) and β′ represents an atomic group necessary for forming a carbocyclic ring, such as a benzene ring, and said benzene ring may be condensed with a carbon ring or a heterocyclic ring to form a naphthalene ring, a quinoline ring, a 5,6,7,8,-tetrahydronaphthalene ring or a chroman ring. Practical examples of Y of this type are described in Japanese Patent Application (OPI) Nos. 113,624/'76; 12,642/'81; 16,130/'81; 16,131/'81; 4043/'82; 650/'82 and US—A—4,053,312.

Furthermore, still another example of Y suitable for the compound of this type is the group represented by formula (CIV)

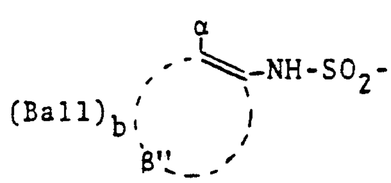

(CIV)

wherein Ball, α, and b have the same meaning as in the case of formula (CII) and β″ represents an atomic group forming a heterocyclic ring such as pyrazole ring or a pyridine ring, said heterocyclic ring may be condensed with a carbon ring of a heterocyclic ring. Practical examples of Y of this type are described in Japanese Patent Application (OPI) No. 104,343/'76.

Furthermore, another example of Y suitable for the compound of this type is the group represented by formula (CV)

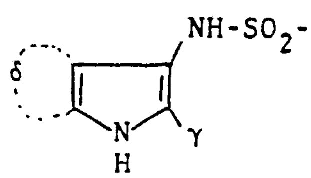

(CV)

wherein γ preferably represents a hydrogen atom, a substituted or unsubstituted alkyl group, a substituted or unsubstituted aryl group, a substituted or unsubstituted heterocylic ring group, or —CO—$G^{21}$ (wherein $G^{21}$ represents —$OG^{22}$, —S—$G^{22}$, or

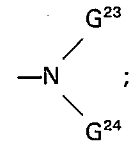

wherein $G^{22}$ represents a hydrogen atom, an alkyl group, a cycloalkyl group, or an aryl group; $G^{23}$ represents a hydrogen atom; an alkyl group, a cycloalkyl group, an aryl group, or an acyl group induced from an aliphatic carboxylic acid, an aromatic carboxylic acid or sulfonic acid; and $G^{24}$ represents a hydrogen atom or an unsubstituted or substituted alkyl group); and δ represents a residue completing a condensed benzene ring.

Practical examples of Y of this kind are described in Japanese Patent Application (OPI) Nos. 104,343/'76; 46,730/'78; 130,122/'79 and 85,055/'82.

Moreover, as Y suitable for the compound of this type, there is the group represented by formula (CVI)

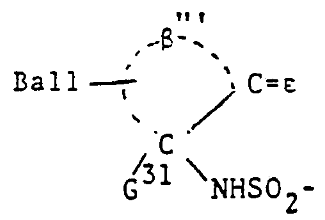

(CVI)

wherein Ball is same as in the case of formula (CII); ε represents an oxygen atom or a $= NG^{32}$ group (wherein $G^{32}$ represents a hydroxy group or an amino group which may have a substituent); in this case, examples of the compound $H_2N—G^{32}$ from which the $= NG^{32}$ group is derived include hydroxylamine, hydrazines, semicarbazides or thiosemicarbazides; β''' represents an atomic group forming a 5-membered, 6-membered or 7-membered saturated or unsaturated non-aromatic hydrocarbon ring; and $G^{31}$ represents a hydrogen atom or a halogen atom such as fluorine, chlorine or bromine. Practical examples of Y of this kind are described in Japanese Patent Application (OPI) Nos. 3819/'78; and 48,534/'79.

Examples of Y of the compound of this type include groups as described, for example, in Japanese Patent Publication Nos. 32,129/'73 and 39,165/'73; Japanese Patent Application (OPI) No. 64,436/'74 and US—A—3,443,934.

As still another example of Y, there is the group represented by formula (CVII)

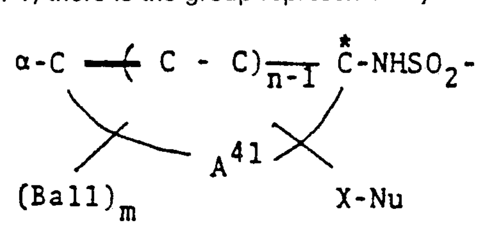

(CVII)

wherein α represents $OR^{41}$ or $NHR^{42}$ (wherein $R^{41}$ represents a hydrogen atom or a hydrolyzable moiety and $R^{42}$ represents a hydrogen atom or an alkyl group having from 1 to 50 carbon atoms); $A^{41}$ represents an atomic group necessary for forming an aromatic ring; Ball represents an organic immobilizing group disposed on an aromatic ring; m represents an integer of 1 or 2, when m is 2, said Ball groups may be the same or different; X represents a divalent organic group having from 1 to 8 carbon atoms and forms a 5- to 12-membered ring with a nucleophilic group (Nu) and an electrophilic center (the carbon atom with *) formed by oxidation; Nu represents a nucleophilic group; n represents an integer of 1 or 2; and α is the same as in the case of above formula (CII). Practical examples of Y of this kind are descrbied in Japanese Patent Application (OPI) No. 20735/'82.

Furthermore, as another type of the compound shown by formula (CI), there is a nondiffusible image-forming compound which causes self ring closure in the presence of a base to release a diffusible dye, but substantially does not release the dye when the compound reacts with the oxidation product of a developing agent.

Examples of Y effective as a compound of this type include groups represented by formula (CVIII)

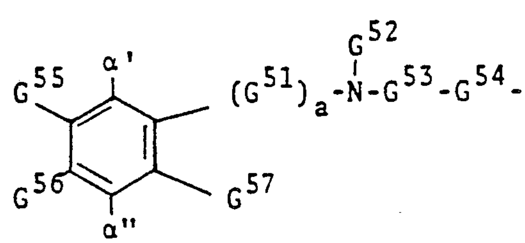

(VIII)

wherein α′ represents an oxidizable nucleophilic group such as a hydroxy group, a primary or secondary amino group, a hydroxyamino group or a sulfonamido group, or a precursor thereof; α″ represents a dialkylamino group or an optional group defined in regard to a α′; $G^{51}$ represents an alkylene group having from 1 to 3 carbon atoms, a represents 0 or 1, $G^{52}$ represents a substituted or unsubstituted alkyl group having from 1 to 40 carbon atoms or a substituted or unsubstituted aryl group having from 6 to 40 carbon atoms, $G^{53}$ represents an electrophilic group such as —CO— or —CS—; and $G^{54}$ represents an oxygen atom, a sulfur atom, a selenium atom or a nitrogen atom, and when $G^{54}$ is a nitrogen atom, it may be substituted by a hydrogen atom, a substituted or unsubstituted alkyl group having from 1 to 10 carbon atoms, or an aromatic residue having 6 to 20 carbon atoms, $G^{55}$, $G^{56}$ and $G^{57}$ each represents a hydrogen atom, a halogen atom, a group containing a carbonyl group (e.g., an amide group and —COOH), a sulfamoyl group a sulfonamide group, an alkyloxy group having from 1 to 40 carbon atoms or represents the same group as defined for $G^{52}$, $G^{55}$ and $G^{57}$ together may form a ring, or $G^{56}$ may be

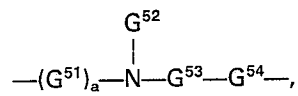

wherein at least one of $G^{52}$, $G^{55}$, $G^{56}$ and $G^{57}$ represents a ballast group. Examples of Y of the compound of this type include groups as described, for example, in Japanese Patent Application (OPI) No. 63,618/′76.

Examples of Y suitable for the compound of this type include groups represented by formulae (CIX) and (CX):

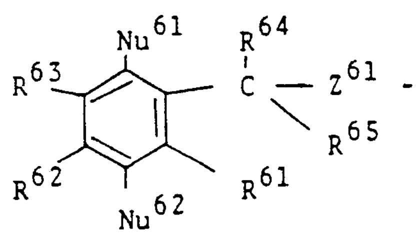

(CIX)

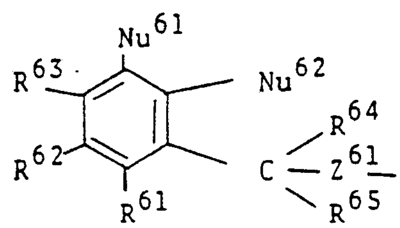

(CX)

wherein $Nu^{61}$ and $Nu^{62}$ (which may be the same or different) each repreents a nucleophilic group or a precursor thereof; $Z^{61}$ represents a divalent atomic group which is electrically negative to the carbon atom to which $R^{64}$ and $R^{65}$ are substituted; $R^{61}$, $R^{62}$, and $R^{63}$ each represents a hydrogen atom, a halogen atom, an alkyl group, an alkoxy group, or an acylamino group; when said $R^{61}$ and $R^{62}$ are adjacently disposed on a ring, they may form a condensed ring with the remaining molecule, or said $R^{62}$ and $R^{63}$ may form a condensed ring with the remaining part of the molecule; $R^{64}$ and $R^{65}$ (which may be the same or different) each represents a hydrogen atom, a hydrocarbon group or a substituted hydrocarbon group; and a sufficiently large ballast group, Ball exists at least on the substituent $R^{61}$, $R^{62}$, $R^{63}$, $R^{64}$, or $R^{65}$ for making the aforesaid compound immobile. Practical examples of Y of this kind are described in Japanese Patent Application (OPI) Nos. 69,033/′78 and 130,927/′79.

Also, another Y suitable for the compound of this type, is the group represented by formula (CXI)

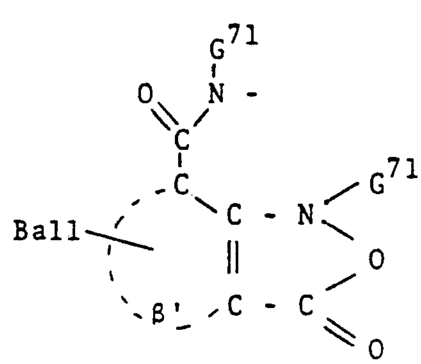

(CXI)

wherein Ball and β' are the same as defined in formula (CIII); $G^{71}$ represents an alkyl group or a substituted alkyl group. Practical examples of Y of this kind are described in Japanese Patent Application (OPI) Nos. 111,628/'74 and 4,810/'77.

Moreover, another example of the compound shown by formula (CI) above is a non-diffusible image-forming compound which does not release a dye by itself, but releases a dye when the compound reacts with a reducing agent. In this case, it is preferred to use a compound mediating the redox reaction (a so-called electron donor) together with the image-forming compound.

As an example of Y effective for the compound of this type, there is a group shown by formula (CXII)

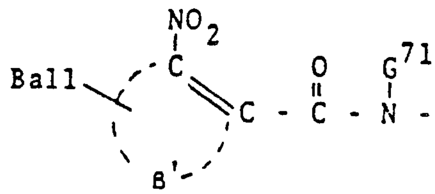

(CXII)

wherein Ball and β' are the same as defined in formula (CIII) and $G^{71}$ represents an alkyl group or a substituted alkyl group. Practical examples of Y of this kind are described in Japanese Patent Application (OPI) Nos. 35,533/'78 and 110,827/'78.

As still another example of Y suitable for the compound of this type, there is the group shown by formula (CXIII)

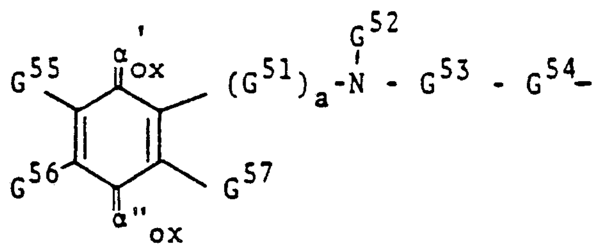

(CXIII)

wherein $\alpha'_{ox}$ and $\alpha''_{ox}$ each represents a group giving α' or α'' by reduction and α', α'', $G^{51}$, $G^{52}$, $G^{53}$, $G^{54}$, $G^{55}$, $G^{56}$, $G^{57}$, and *a* are same as in the case of formula (VIII). Practical examples of Y of this kind are described in Japanese Patent Application (OPI) No. 110,827/'78 and U.S. Patents 4,356,249 and 4,358,525.

Other examples of Y suitable for the compound of this type include groups represented by formulae (CXIV A) and (CXIV B)

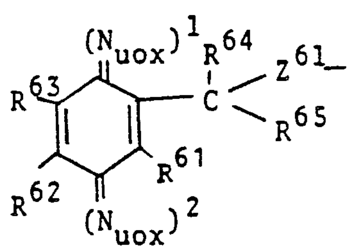

(CXIV A)

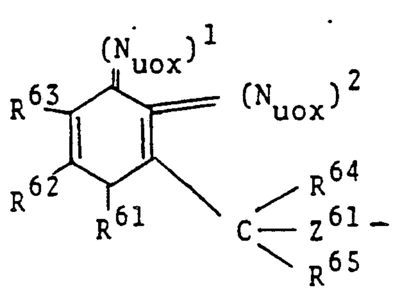

(CXIV B)

wherein $(N_{uox})^1$ and $(N_{uox})^2$, which may be the same or different, each represents an oxidized nucleophilic group and the other symbols are the same as in the cases of formulae (CIX) and (CX). Practical examples of Y of this kind are described in Japanese Patent Application (OPI) Nos. 130,927/'79 and 164,342/'81.

In the patent specifications cited in regard to formulae (CXII), (CXIII), (CXIV A) and (CXIV B), electron donors which can be used with the aforesaid compounds are described.

Also, as another type of compound shown by general formula (I), there is further a linked donor acceptor compound (LDA compound). This compound is a non-diffusible image-forming compound which causes a donor-acceptor reaction in the presence of a base to release a diffusible dye, but does not substantially release the dye when the compound reacts with the oxidation product of a developing agent.

An example of Y effective for the compound of this type is, for example, the group shown by the following formula (CXV). Practical examples of Y of this kind are described in GB—A—2,140,927.

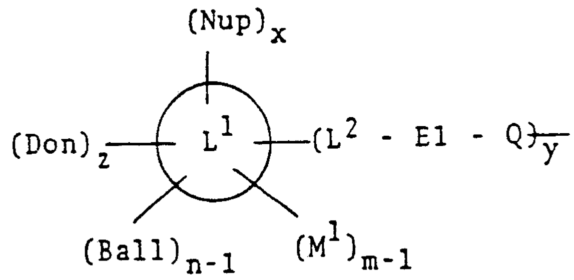

(CXV)

wherein n, x, y, and z are 1 or 2; m represents an integer of 1 or more; Don represents a group containing an electron donor or a precursor moiety thereof; $L^1$ represents an organic group connecting Nup and —$L^2$—E1—Q or Don; Nup represents a precursor for a nucleophilic group; E1 represents an electrophilic center; Q represents a divalent group; Ball represents a ballast group; $L^2$ represents a linkage group and $M^1$ represents an optional substituent.

The ballast group is an organic ballast group capable of rendering the dye image-forming compound non-diffusible, and it is preferred that the ballast group is a group containing a hydrophobic group having from 8 to 32 carbon atoms. Such an organic ballast group is bonded to the dye image-forming compound directly or through a linkage group (e.g., an imino bond, an ether bond, a thioether bond, a carbonamido bond, a sulfonamido bond, a ureido bond, an ester bond, an imido bond, a carbamoyl bond or a sulfamoyl bond), either singly or as a combination thereof.

The dye-providing materials may be used solely or in a combination of two or more. The case of using a mixture, the same color may be formed by using two or more dyes, or black may be formed using two or more dyes.

Practical examples of the image-forming material for use in this invention are described in the patent specifications indicated above. Exemplary compounds are shown below.

For example, the dye-providing material shown by foregoing formula (CI) are as follows.

(1)

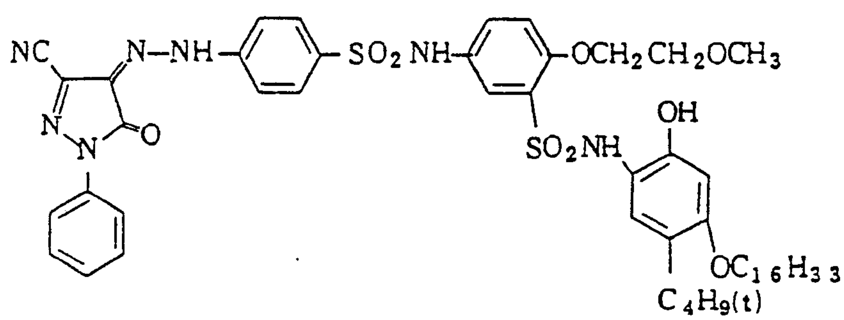

(2)

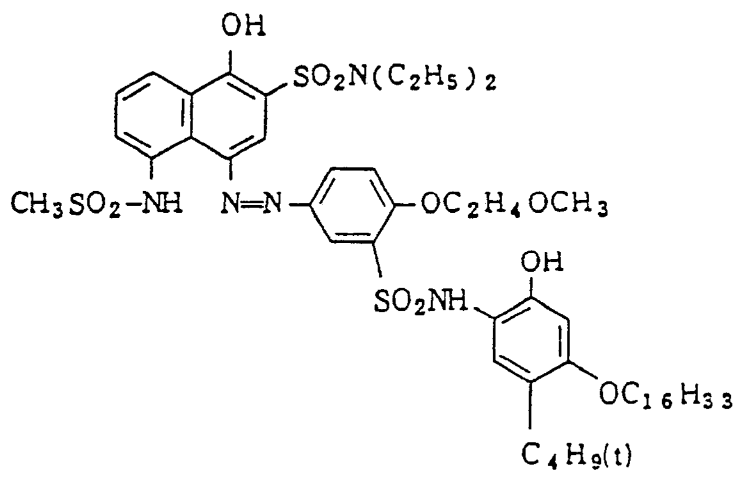

(3)

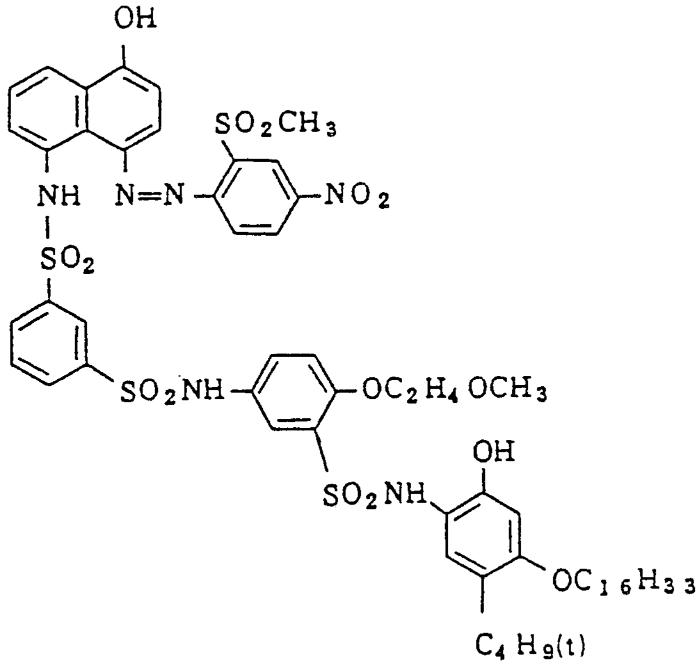

(4)

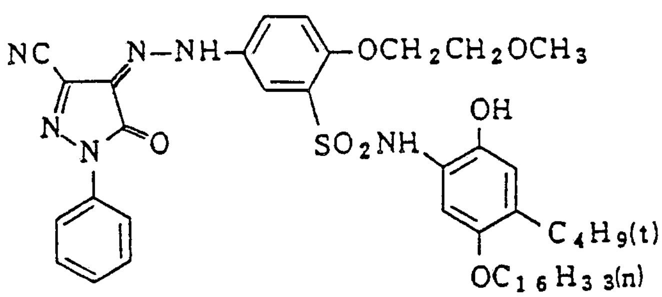

(5)

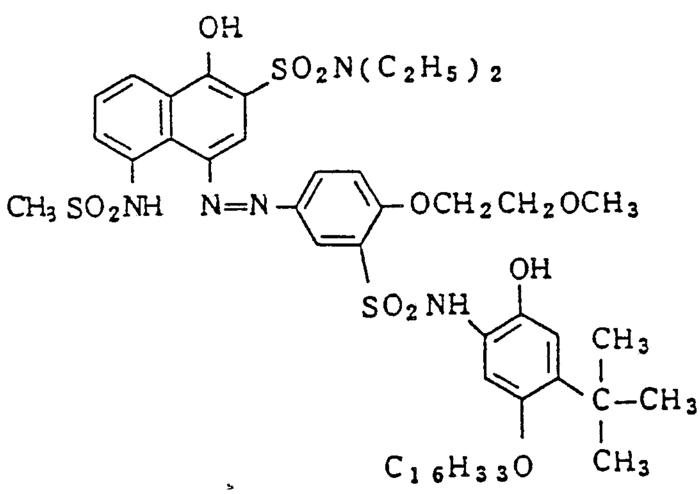

(6)

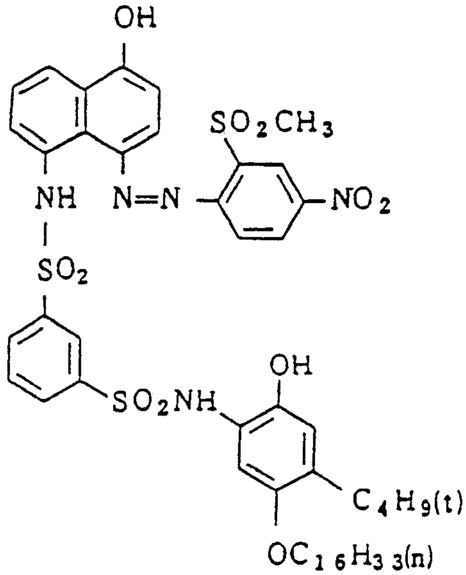

(7)

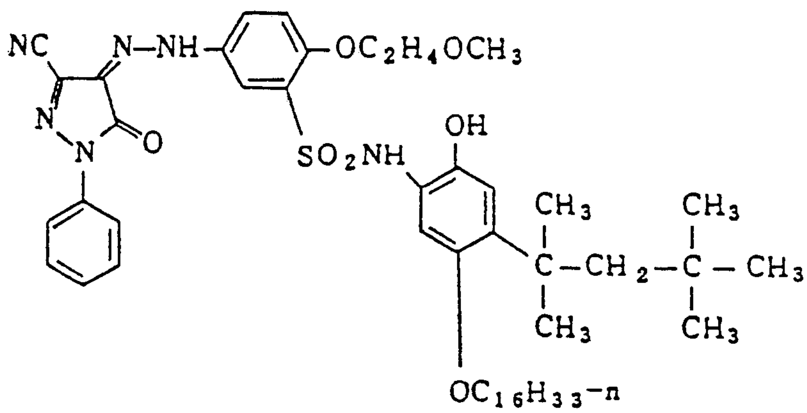

(8)

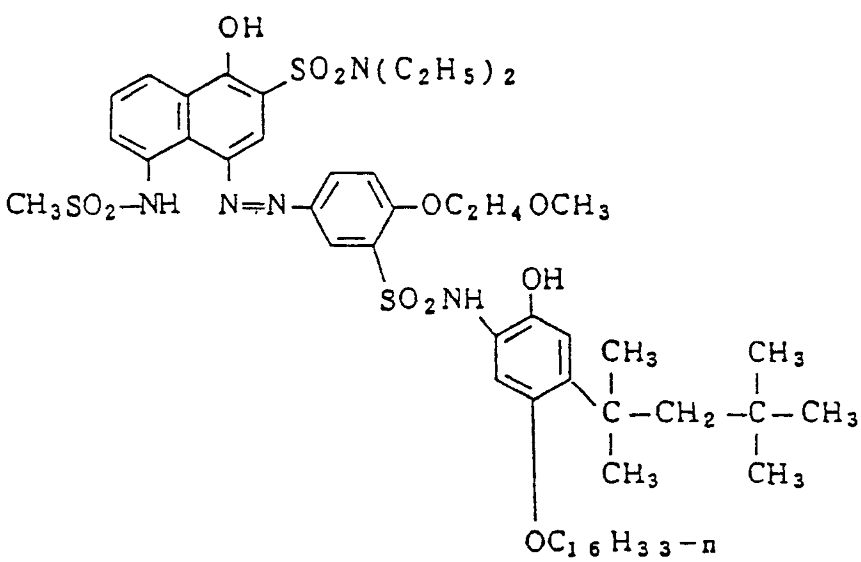

(9)

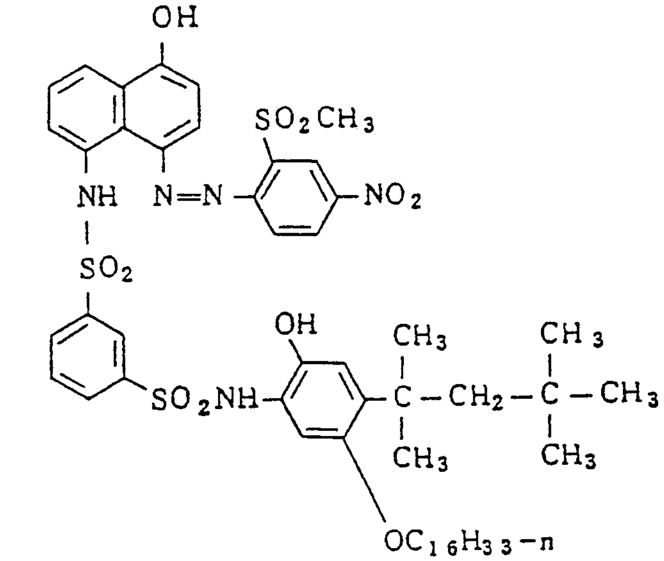

(10)

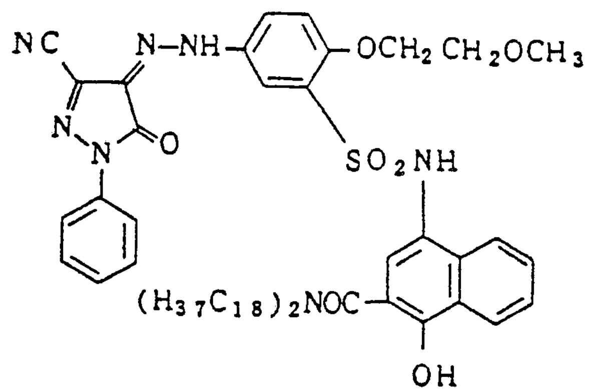

(11)

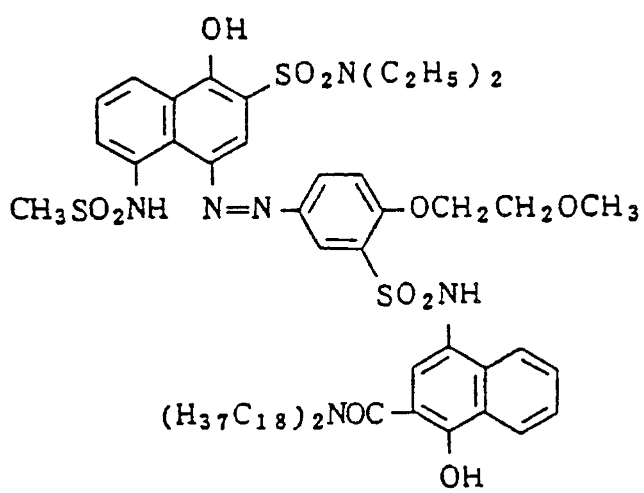

(12)

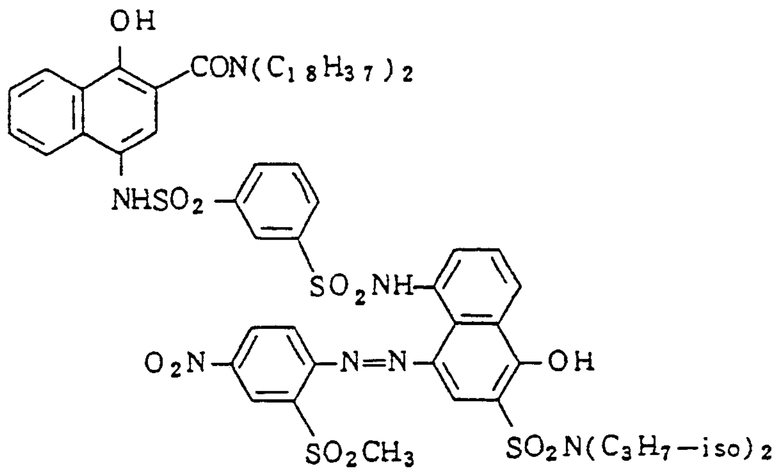

(13)

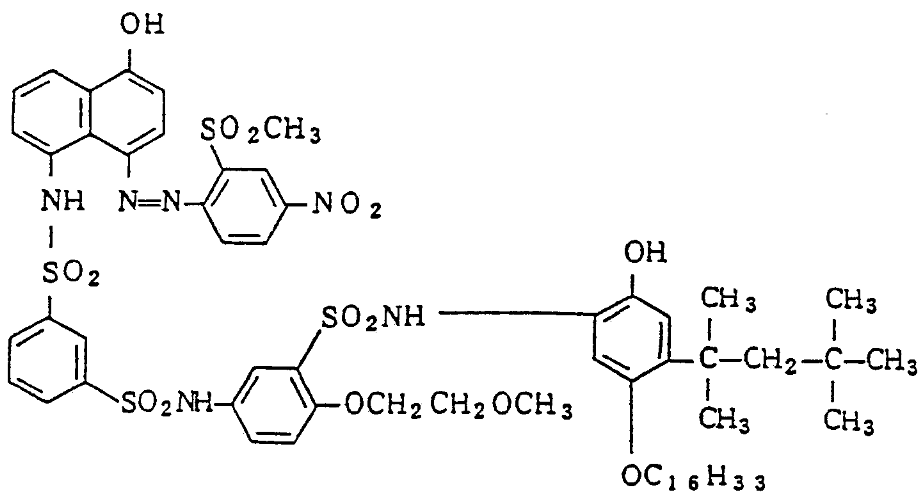

(14)

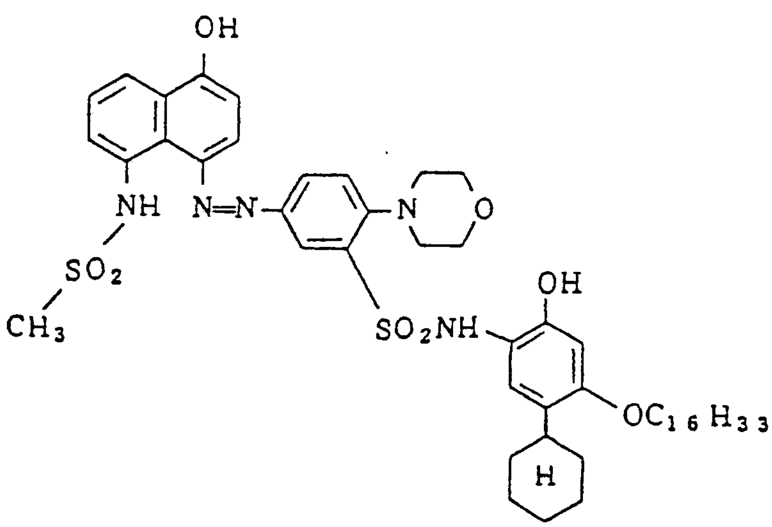

(15)

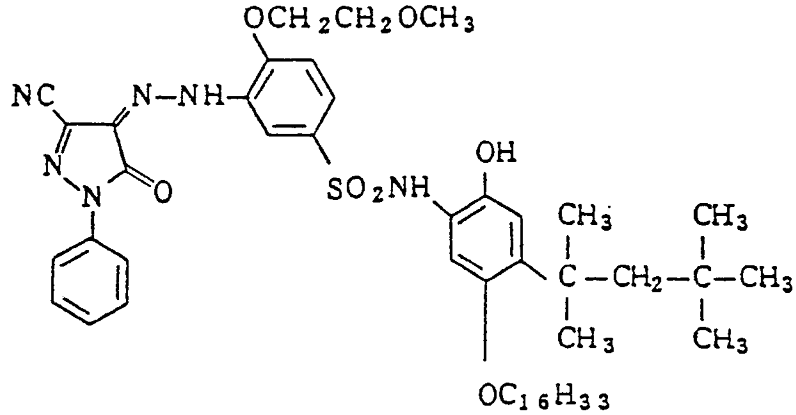

The above-described specific compounds are illustrated as non-limiting examples of the dye-providing materials for use in this invention.

In the present invention a dye-providing material which releases or produces a mobile dye corresponding to or reversely corresponding to a reduction reaction of a silver halide through a reducing agent may also be preferably used. Examples of such materials include a coupler, a dye which is able to form positive color images by a photographic silver dye bleaching process, a dye having introduced therein a nitrogen-containing heterocyclic ring group, a dye-providing material which releases a mobile dye by a coupling reaction with a reducing agent which is oxidized by an oxidation reduction reaction with a silver halide or an organic silver salt upon heat development, a non-diffusible image-forming compound which causes self ring closure in the presence of a base to release a diffusible dye, but does not release the dye when the compound reacts with the oxidation product of a developing agent, a non-diffusible image-forming compound which does not release a dye by itself but releases a dye when the compound reacts with a reducing agent, and a linked donor acceptor compound which is a nondiffusible image-forming compound and causes a donor-acceptor reaction in the presence of a base to release a diffusible dye, but does not substantially release the dye when the compound reacts with the oxidation product of a developing agent.

Many of the above-described materials form an imagewise distribution of a mobile dye by heat development corresponding to the exposed portion of a light-sensitive material, and a process for visualizing the dye image in a dye-fixing material (so-called diffusion transfer process) is described in the above-indicated patent specifications and EP—A—119,615.

In the present invention, the dye providing material and other photographic additives may be introduced to the layer of the light sensitive material according on the method disclosed in column 13 of US—A— .

In the present invention, developing agents and reducing agents as disclosed in column 49 of US—A—4,500,626 may be used.

In the present invention, silver halide and organic silver compounds as disclosed in columns 14—15 in US—A—4,500,627 may be used. Binders which are disclosed in column 15 of the same patent may also be used in the present invention.

When spectral sensitization is carried out in the present invention sensitizing dyes disclosed in columns 15—16 of US—A—4,500,627 may be used in the manner according on the method disclosed therein.

Methods for exposure, development and after-treatment disclosed in column 20 of US—A—4,500,627 may be applied to the heat developable light-sensitive material of the present invention.

Supports, surfactants, hardening agents, mordants, dye transfer assistants, dye fixing layers, dye fixing materials, etc., which are disclosed in US—A—4,500,627 may be used in the present invention.

Example 1

Preparation of Silver Iodobromide Emulsion

Gelatin (40 g) and KBr (26 g) were dissolved in water (3,000 ml). The solution was agitated at 50°C. A solution obtained by dissolving silver nitrate (34 g) in water (200 ml), and a solution (200 ml) obtained by dissolving dye I (0.02 g) shown hereinafter were added to the KBr solution over a period of 10 minutes. To this solution, a solution of KI (3.3 g) in water (100 ml) was added over a period of 2 minutes. The pH of the thus prepared silver idobromide emulsion was adjusted to precipitate the emulsion and the excess salt was then filtered out. The pH of the emulsion was adjusted to 6.0 to obtain a silver iodrobomide emulsion (yield: 400 g).

Preparation of Coupler Dispersion in Gelatin

2-dodecylcarbamoyl-1-naphthol (dye providing material (17) 5 g), succinic acid-2-ethylhexyl ester sodium sulfonate (0.5 g) and tricresyl phosphate (TCP) (2.5 g) were dissolved in ethyl acetate (30 ml). The resulting solution was mixed with a 10 wt% gelatin solution (100 g) under agitation, and, the mixture was dispersed using a homogenizer for 10 minutes at 10,000 rpm.

A coating liquid having the composition indicated below was applied to a polyethylene terephthalate film base to give a wet thickness of 60 µm and then dried to prepare a light-sensitive material.

| | | |
|---|---|---|
| (a) | Silver iodobromide emulsion | 10 g |
| (b) | Coupler dispersion in gelatin | 3.5 g |
| (c) | Solution of base precursor (1) of the present invention | 0.28 g |
| (d) | Gelatin (10 wt% aq. sol.) | 5 g |
| (e) | Solution of 0.2 g of 2,6-dichloro-p-aminophenol dissolved in 17 ml of water. | |

The light-sensitive material thus prepared was imagewise exposed using a tungsten lamp (2,000 lux) for 5 seconds. Then, the exposed material was heated uniformly on a heat block (150°C, 20 sec) to provide a negative cyan dye image. The image density was measured with a Macbeth transmission densitometer (Model TD-504): Dmin was 0.26 and Dmax was 2.13.

The above result indicates that the base precursor according to the present invention provides a high density.

Dye I

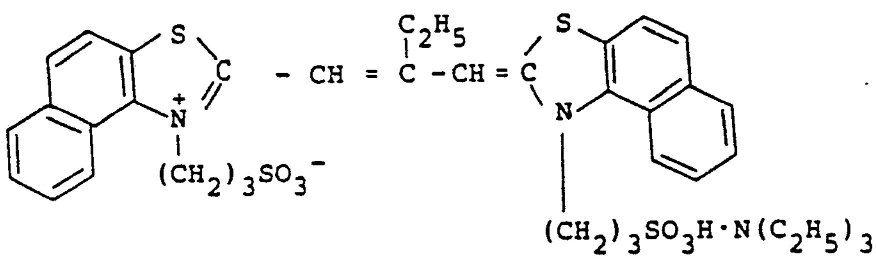

Example 2

In this example, a silver iodobromide emulsion of the same type as used in Example 1, and a dispersion of dye releasing material prepared as follows were used.

Preparation of Dispersion of Dye Releasing Material

Five grams of a dye releasing material (2) and 0.5 g of surfactant i.e. succinic acid-2-ethylhexyl ester sodium sulfonate and 5 g of tricresyl phosphate (TCP) were dissolved in 30 ml of ethyl acetate under heating at about 60°C. The resulting solution was mixed with a 10 wt% gelatin solution (100 g) under agitation, and the resulting mixture was dispersed using a homogenizer for 10 minutes at 10,000 rpm.

A light-sensitive composition was prepared from the following formulation.

| | | |
|---|---|---|
| (a) | Light-sensitive silver iodobromide emulsion (as shown in Example 1) | 25 g |
| (b) | Dispersion of dye releasing material (2) | 33 g |
| (c) | 5 wt% Aqueous solution of the following compound | 10 ml |

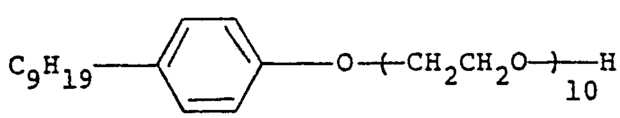

| | | |
|---|---|---|
| (d) | 10 wt% Aqueous solution of the following compound: | 4 ml |
| | $H_2NSO_2N(CH_3)_2$ | |
| (e) | Base precursor (1) of the present invention | 2.7 g |
| (f) | Water | 20 ml |

The above components of (a) ~ (f) were mixed and dissolved under heating. The resulting solution was applied onto a polyethylene terephthalate film base to give a wet thickness of 30 μm and then dried to provide a light-sensitive material. This material was imagewise exposed using a tungsten lamp (2,000 lux) for 10 seconds and heated uniformly on a heat block (150°C) for 20 seconds to provide sample A.

Sample B was prepared by following the same procedure as above except that 1.8 g of guanidinetrichloroacetic acid was used instead of the compound of this invention of (e) in Sample A, Sample C was prepared using 2.1 g of guanidine phenylsulfonylacetate instead of the aforesaid compound of this invention, and Sample D was prepared using 2,2 g of guanidine 3-sulfamoylphenylsulfonylacetate instead of the aforesaid compound of this invention and these samples were processed by the same manner as above.

Preparation of Image-receiving Material

10 g of methyl acrylate-N,N,N-trimethyl-N-vinyl-benzylammonium chloride copolymer (molar ratio of methyl acrylate to vinyl benzyl ammonium chloride is 1:1) was dissolved in water (200 ml), and the solution was mixed uniformly with 10 wt% lime-treated gelatin (100 g). The resulting mixture was uniformly spread onto a paper base laminated with $TiO_2$ dispersed polyethylene, thereby forming an image-receiving layer of a uniform wet thickness of 90 μm. The layer was dried to provide an image-receiving material.

The image-receiving material was dipped in water and recovered therefrom. Samples A, B, C and D of the light-sensitive material heated were superimposed on each sample of image-receiving material in such a manner that each of the light-sensitive layers was in contact with the image-receiving layer, respectively.

After heating on a heat block (80°C) for 6 seconds, each image-receiving material was peeled from each light-sensitive material. A negative magenta image was formed on each image-receiving material. The density of each negative image was measured with a Macbeth (RD-519) reflection densitometer. The results were as follows.

| Sample No. | Dmax | Dmin |
|---|---|---|
| A (Present Invention) | 1.95 | 0.15 |
| B (Control) | 2.14 | 0.58 |
| C (Control) | 1.28 | 0.16 |
| D (Control) | 1.45 | 0.15 |

The above results show that the base precursor according to the present invention gives high maximum and low minimum densities.

Samples A, B, C and D were left to stand at 60°C for 2 days, and treated as above. The Dmin and Dmax of the image of Sample A were 0.23 and 2.01, Sample C were 0.20 and 1.33 and Sample D were 0.27 and 1.49 respectively, but fog occurred throughout the surface of sample B. Thus, the Sample of the present invention has an improved storage stability.

Example 3

The procedure of Example 2 was repeated except that the base precursors shown in the following table were used. The results are also shown in the same table.

| Sample No. | Base Precursor | (g) | Dmax |
|---|---|---|---|
| D | Compound (2) | 2.8 | 2.03 |
| E | Compound (6) | 2.8 | 2.03 |
| E | Compound (6) | 2.5 | 1.79 |
| F | Compound (9) | 3.0 | 1.92 |
| G | Compound (15) | 3.2 | 2.00 |
| H | Compound (16) | 3.3 | 2.12 |
| I | Compound (20) | 3.8 | 1.82 |
| J | Compound (22) | 3.5 | 1.99 |

The above results show that base precursors according to the present invention give high maximum densities.

Example 4

In this example, an organic silver salt oxidizing agent was used.

Preparation of Silver Benzotriazole Emulsion

Gelatin (28 g) and benzotriazole (13.2 g) were dissolved in water (3,000 ml). The resulting solution was agitated at 40°C. To this solution, a solution having silver nitrate (17 g) dissolved in water (100 ml) was added over a period of 2 minutes.

The resulting benzotriazole silver emulsion was pH-adjusted to precipitate, and the excess salt was filtered out. The emulsion was adjusted to a pH of 6.0, thereby providing a silver benzotriazole emulsion (yield: 400 g).

Using this silver benzotriazole emulsion, a light-sensitive coating composition was prepared from the following formulation.

| | | |
|---|---|---|
| (a) | Silver iodobromide emulsion (as prepared in Example 1) | 20 g |
| (b) | Silver benzotriazole emulsion | 10 g |
| (c) | Dispersion of dye releasing material (as prepared in Example 2) | 33 g |
| (d) | 5% Aqueous solution of the following compound: | 10 ml |

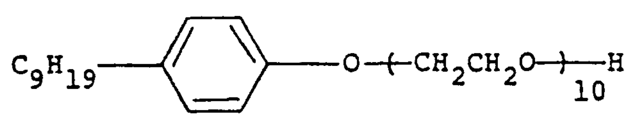

| | | |
|---|---|---|
| (e) | 10% Aqueous solution of the following compound: | 4 ml |

$$H_2NSO_2N(CH_3)_2$$

| | | |
|---|---|---|
| (f) | Base precursor (1) of the present invention | 3 g |
| (g) | Gelatin dispersion of the acid precursor shown below | 8 ml |
| (h) | Water | 12 ml |

The gelatin dispersion of the acid precursor of the aforesaid component (g) was prepared as follows.

To 100 g of 1% aqueous solution of gelatin was added 10 g of the compound shown below and the compound was pulverized for 10 minutes in a mill using 100 g of glass beads having a mean particle size of about 0.6 mm. By separating the glass beads by filtration, a gelatin dispersion of the acid precursor was obtained.

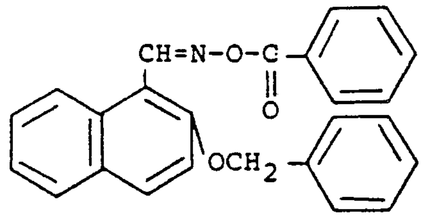

Above components (a) to (g) were mixed and by following the same procedure as in Example 2 using the mixture, Samples (A'), (B') and (C') were prepared. The samples were also processed as in Example 2 and the results thus obtained are shown below.

| | Sample | Dmax | Dmin |
|---|---|---|---|
| (A') | Containing Base Precursor (1) (This invention) | 2.19 | 0.16 |
| (B') | Containing Guanidine-trichloroacetic Acid (Comparison) | 2.33 | 0.61 |
| (C') | Containing Guanidine Phenylsulfonyl-acetate (Comparison) | 1.47 | 0.19 |

From the results, it can be seen that the sample of this invention containing the base precursor of this invention gives both a high maximum density and a low minimum density.

Furthermore, after storing Samples (A'), (B') and (C') for 2 days at 60°C, and then processing in the same manner as above the results showed that the minimum density and the maximum density were 0.23 and 2.21, respectively for Sample (A') and 0.20 and 1.52, respectively for Sample (C'), while Sample (B') was overall fogged. Thus, it can be seen that the sample of this invention shows a good storage stability.

Example 5

Preparation of Silver Benzotriazole Emulsion Containing Light-sensitive Silver Bromide

Benzotriazole (6.5 g) and gelatin (10 g) were dissolved in water (1,000 ml). The resulting solution was agitated at 50°C. To this solution, a solution of silver nitrate (8.5 g) dissolved in water (100 ml) was added over a period of 2 minutes.

Then, a solution of potassium bromide (1.2 g) dissolved in water (50 ml) was added to the above-obtained solution over a period of 2 minutes. The thus prepared emulsion was pH-adjusted to precipitate, and the excess salt was filtered out. The emulsion was adjusted to a pH of 6.0, thereby providing a silver benzotriazole emulsion (yield: 200 g).

Preparation of Gelatin Dispersion of Dye Releasing Material (16)

10 g of a dye releasing material (16) of the following formula:

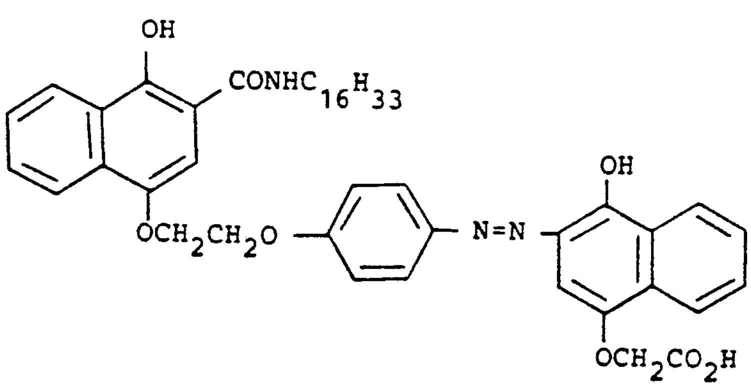

and 0.5 g of a surfactant i.e., succinic acid-2-ethylhexyl ester sodium sulfate, and 4 g of tricresyl phosphate (TCP) were dissolved in 20 ml of cyclohexanone under heating at about 60°C, thereby producing a uniform

solution. This solution was mixed with a 10 wt% solution of lime-treated gelatin (100 g) under agitation, and the mixture was dispersed with a homogenizer for 10 minutes at 10,000 rpm.

A coating composition for light-sensitive material was prepared from the following formulation

| | | |
|---|---|---|
| (a) | Silver benzotriazole emulsion containing light-sensitive silver bromide | 10 g |
| (b) | Dispersion of dye releasing material | 3.5 g |
| (c) | Base precursor (1) of the present invention | 0.28 g |
| (d) | Gelatin (10 wt% aq. sol.) | 5 g |
| (e) | Solution having 2,6-dichloro-4-amino-phenol (200 ml) dissolved in methanol (4 ml) | |

The above components (a)—(e) were mixed and dissolved under heating. The resulting solution was applied to a polyethylene terephthalate film base (180 μm thick) to form a light-sensitive layer having a wet thickness of 30 μm. The resulting web was dried and imagewise exposed using a tungsten lamp (2000 lux) for 10 seconds and subsequently heated uniformly on a heat block (150°C) for 20 seconds.

The heated sample of light-sensitive material was superimposed on an image-receiving material prepared in Example 2, and subsequently processed as in Example 2 to provide a negative magenta image on the image-receiving material. Measurement with a reflection densitometer showed that the negative image had a Dmax 2.06 and a Dmin 0.20.

Thus it was found that the compound of the present invention provides excellent effects.

Example 6

Preparation of Gelatin Dispersion of Dye Releasing Material (18)

5 g of each of reducible dye releasing materials of

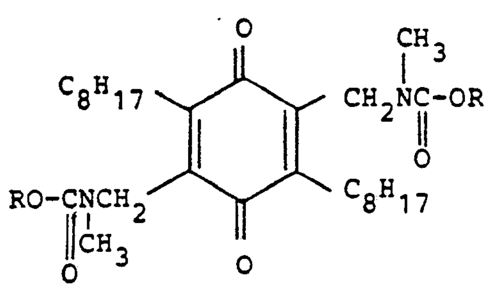

and

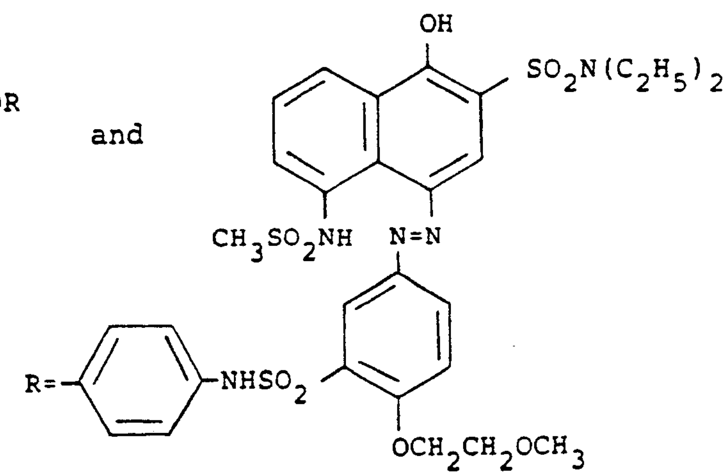

4 g of the electron donor having the following formula:

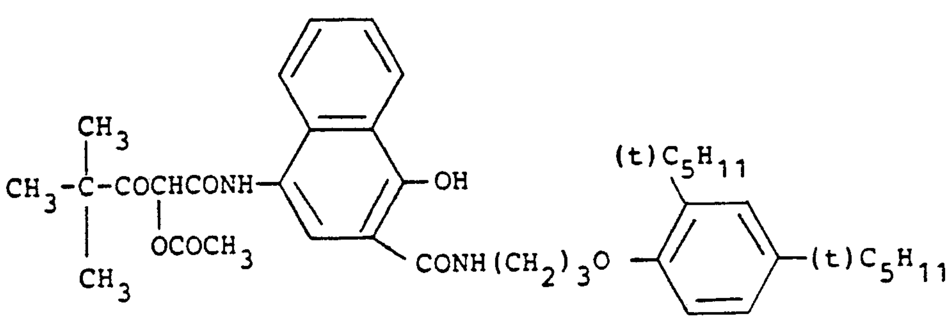

and 0.5 g of succinic acid-2-ethylhexyl ester sodium sulfate, and 10 g of tricresyl phosphate (TCP) were dissolved in 20 ml of cyclohexanone under heating at about 60°C. This solution was mixed with a 10 wt% solution of gelatin (100 g) under agitation, and the mixture was dispersed with a homogenizer for 10 minutes at 10,000 rpm.

A coating composition for light-sensitive material was prepared from the following formulation.

| | | |
|---|---|---|
| (a) | Silver benzotriazole emulsion containing light-sensitive silver bromide (obtained in Example 5) | 10 g |
| (b) | Dispersion of dye releasing material (obtained in this Example) | 3.5 g |
| (c) | Base precursor (1) of the present invention | 0.4 g |
| (d) | 5% Aqueous solution of the following compound: | 1.5 ml |

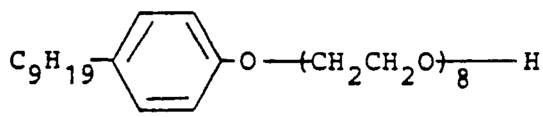

To the above components (a)—(d) 4 ml of water were added, mixed and dissolved under heating. The resulting solution was applied to a polyethylene terephthalate film base to form a light-sensitive layer having a wet thickness of 30 μm. The resulting web was dried and imagewise exposed using a tungsten lamp (2000 lux) for 10 seconds and subsequently heated uniformly on a heat block (140°C) for 40 seconds.

The heated sample of light-sensitive material was superimposed on an image-receiving material prepared in Example 2 previously impregnated with water, and subsequently processed as in Example 2 to provide a positive magenta image on the image-receiving material. Measurement with a reflection densitometer using green light showed that the image had a Dmax 1.92 and a Dmin 0.26.

Thus it was found that the base precursor of the present invention provides excellent effects.

**Claims**

1. A heat developable light-sensitive material comprising a support and formed thereon a silver halide emulsion layer, wherein said light-sensitive material contains at least one compound selected from the group consisting of compounds represented by formula (I) or (II) as a base precursor:

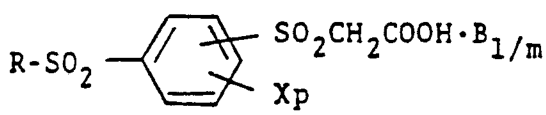
(I)

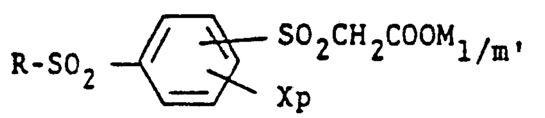
(II)

wherein

R represents a substituted or unsubstituted alkyl group, a substituted or unsubstituted cycloalkyl group, a substituted or unsubstituted alkenyl group, a substituted or unsubstituted alkynyl group, a substituted or unsubstituted aralkyl group, a substituted or unsubstituted aryl group or a heterocyclic group provided that R is not carboxymethyl;

X represents a group or atom selected from the group consisting of a hydrogen atom, a halogen atom, an alkyl group, a cycloalkyl group, an aralkyl group, an alkoxy group, an aryloxy group, an aryl group, an alkyl or aryl acylamino group, an alkyl or aryl acyl group, a cyano group, an alkylsulfonylamino group, a nitro group, an arylsulfonylamino group, an alkylsulfonyl group, an arylsulfonyl group, a sulfamoyl group, a substituted sulfamoyl group, a carbamoyl group, a substituted carbamoyl group, an alkylthio group, an arylthio group, an alkoxycarbonyl group, an aryloxycarbonyl group, an alkyl or arylacyloxy group, and substituted groups thereof at the alkyl or aryl moiety thereof, $-OM_{1/m'}$, $-COOM_{1/m'}$, $-OH\cdot B_{1/m}$ (wherein $-OH$ is phenolic) and $-COOH\cdot B_{1/m'}$;

p represents an integer of 0 to 4;

B represents a monoacidic base or a diacidic base;

M represents an alkali metal or an alkaline earth metal; and

m represents 1 when B represents a monoacidic base and represents 2 when B represents a diacidic base, and m' represents the valence number of M.

2. The heat developable light-sensitive material of claim 1, wherein the heterocyclic ring represented by R is a 5- or 6-membered heterocyclic ring containing at least one of nitrogen atom, an oxygen atom, and a sulfur atom.

3. The heat developable light-sensitive material of claim 2, wherein said heterocyclic ring is selected from the group consisting of a pyridyl group, pyrazolyl group, and imidazolyl group.

4. The heat developable light-sensitive material of claim 1, wherein the substituent of said substituted groups represented by R is selected from the group consisting of an alkyl group, an alkyl- or arylsulfonyl group, a sulfamoyl group, a N-alkyl- or N-arylsulfamoyl group, a carbamoyl group, a N-alkyl- or N-arylcarbamoyl group, an alkyl or arylsulfonamido group, an alkyl- or arylacyl amido group, a halogen atom, and a hydroxycarbonyl group.

5. The heat developable light-sensitive material of claim 1, wherein B is an organic acid base having a pKa of not less than 7 and having not more than 12 carbon atoms.

6. The heat developable light-sensitive material of claim 5, wherein B has a boiling point of not less than 150°C.

7. The heat developable light-sensitive material of claim 6, wherein B is a monoacidic or diacidic nitrogen containing sulfur free base.

8. The heat developable light-sensitive material of claim 5, wherein B represents an organic base selected from the group consisting of an aromatic or aliphatic amine, an aromatic or aliphatic diamine, a piperidine, piperazine, a guanidine, a cyclic guanidine, an amidine, a cyclic amidine and a tetraalkylammonium hydroxide.

9. The heat developable light-sensitive material of claim 1, wherein M represents an atom selected from the group consisting of Na, K, Cs and Ba.

10. The heat developable light-sensitive material of claim 1, wherein said base precursor is incorporated in an amount of from 0.001 to 50% by weight based on the total weight of the dry coated layer of the light-sensitive material.

11. The heat developable light-sensitive material of claim 1, wherein said silver halide emulsion contains a sensitizing dye.

12. The heat developable light-sensitive material of claim 11 wherein the amount of the sensitizing dye is from 0.001 to 20 g per 100 g of silver of the silver halide emulsion.

13. The heat developable light-sensitive material of claim 1 wherein the light-sensitive material contains a dye providing material which releases or produces a mobile dye corresponding to or reversely corresponding to a reduction reaction of a silver halide through a reducing agent as a image forming material.

14. The heat developable light-sensitive material of claim 1, wherein the light-sensitive material contains at least one image forming material selected from the group consisting of a coupler, a dye which is able to form positive color images by a photographic silver dye bleaching process, a dye having introduced therein a nitrogen-containing heterocyclic ring group, a dye-providing material which releases a mobile dye by a coupling reaction with a reducing agent which is oxidized by an oxidation reduction reaction with a silver halide or an organic silver salt upon heat development, a non-diffusible image-forming compound which causes self ring closure in the presence of a base to release a diffusible dye, but does not release the dye when the compound reacts with the oxidation product of a developing agent, and a non-diffusible image-forming compound which does not release a dye by itself but releases a dye when the compound reacts with a reducing agent, and a linked donor acceptor compound which is a nondiffusible image-forming compound and causes a donor-acceptor reaction in the presence of a base to release a diffusible dye, but does not substantially release the dye when the compound reacts with the oxidation product of a developing agent.

15. The heat developable light-sensitive material of claim 14, wherein the light-sensitive material further contains a dye-providing material which releases a mobile dye as a result of an oxidation-reduction reaction with silver halide or an organic silver salt upon heat development.

16. The heat developable light-sensitive material of claim 1 wherein the light-sensitive material further comprises at least one of an interlayer, a filter layer, an antihalation layer, a protective layer, and an image fixing layer provided on the support.

17. The heat developable light-sensitive material of claim 1, wherein said base precursor is incorporated in the silver halide emulsion layer.

18. The heat developable light-sensitive material of claim 1, wherein the light-sensitive material further comprises an image fixing layer provided on a support other than that for the light-sensitive layer, and said base precursor is incorporated in the image fixing layer.

19. A method for producing an image, which comprises heat developing a heat developable light-sensitive material according to any of claims 1 to 18.

**Patentansprüche**

1. Wärmeentwickelbares lichtempfindliches Material, umfassend einen Träger und eine darauf gebildete Silberhalogenidemulsionsschicht, wobei das lichtempfindliche Material wenigstens eine Verbindung, gewählt aus der Gruppe, bestehend aus Verbindungen der Formel I oder II, als Basenvorläufer enthält:

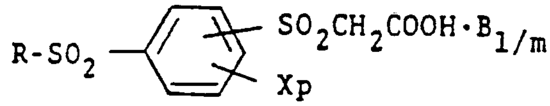 (I)

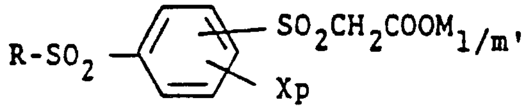 (II)

worin

R eine substituierte oder unsubstituierte Alkylgruppe, eine substituierte oder unsubstituierte Cycloalkylgruppe, eine substituierte oder unsubstituierte Alkenylgruppe, eine substituierte oder unsubstituierte Alkynylgruppe, eine substituierte oder unsubstituierte Aralkylgruppe, eine substituierte oder unsubstituierte Arylgruppe oder eine heterocyclische Gruppe bedeutet mit der Maßgabe, daß R nicht Carboxymethyl ist;

X eine Gruppe oder ein Atom, gewählt aus der Gruppe, bestehend aus einem Wasserstoffatom, einem Halogenatom, einer Alkylgruppe, einer Cycloalkylgruppe, einer Aralkylgruppe, einer Alkoxygruppe, einer Aryloxygruppe, einer Arylgruppe, einer Alkyl- oder Arylacylaminogruppe, einer Alkyl- oder Arylacylgruppe, einer Cyanogruppe, einer Alkylsulfonylaminogruppe, einer Nitrogruppe, einer Arylsulfonylaminogruppe, einer Alkylsulfonylgruppe, einer Arylsulfonylgruppe, einer Sulfamoylgruppe, einer substituierten Sulfamoylgruppe, einer Carbamoylgruppe, einer substituierten Carbamoylgruppe, einer Alkylthiogruppe, einer Arylthiogruppe, einer Alkoxycarbonylgruppe, einer Aryloxycarbonylgruppe, einer Alkyl- oder Arylacyloxygruppe und substituierten Gruppen davon an dem Alkyl- oder Arylrest davon, $—OM_{1/m'}$, $—COOM_{1/m'}$, $—OH \cdot B_{1/m}$ (worin —OH phenolisch ist) und $—COOH \cdot B_{1/m'}$ bedeutet;

p eine ganz Zahl von 0 bis 4 bedeutet;

B eine monoacidische Base oder eine diacidische Base bedeutet;

M ein Alkalimetall oder ein Erdalkalimetall bedeutet; und

m 1 bedeutet, wenn B eine monoacidische Base bedeutet, und 2 bedeutet, wenn B eine diacidische Base bedeutet, und m' die Wertigkeit von M bedeutet.

2. Wärmeentwickelbares lichtempfindliches Material nach Anspruch 1, worin der heterocyclische Ring, dargestellt durch R, ein 5- oder 6-gliedriger heterocyclischer Ring ist, der wenigstens ein Atom aus einem Stickstoffatom, einem Sauerstoffatom und einem Schwefelatom enthält.

3. Wärmeentwickelbares lichtempfindliches Material nach Anspruch 2, worin der heterocyclische Ring aus der Gruppe, bestehend aus einer Pyridylgruppe, Pyrazolylgruppe und Imidazolylgruppe, gewählt wird.

4. Wärmeentwickelbares lichtempfindliches Material nach Anspruch 1, worin der Substituent der substituierten Gruppen, dargestellt durch R, aus der Gruppe, bestehend aus einer Alkylgruppe, einer Alkyl- oder Arylsolfonylgruppe, einer Sulfamoylgruppe, einer N-Alkyl- oder N-Arylsulfamoylgruppe, einer Carbamoylgruppe, einer N-Alkyl- oder N-Arylcarbamoylgruppe, einer Alkyl- oder Arylsulfonamidogruppe, einer Alkyl- oder Arylacylamidogruppe, einem Halogenatom und einer Hydroxycarbonylgruppe, gewählt wird.

5. Wärmeentwickelbares lichtempfindliches Material nach Anspruch 1, worin B eine Base einer organischen Säure mit einem pKa von nicht weniger als 7 und nicht mehr als 12 Kohlenstoffatomen ist.

6. Wärmeentwickelbares lichtempfindliches Material nach Anspruch 5, worin B einen Siedepunkt von nicht weniger als 150°C besitzt.

7. Wärmeentwickelbares lichtempfindliches Material nach Anspruch 6, worin B eine monoacidische oder diacidische stickstoffhaltige schwefelfreie Base ist.

8. Wärmeentwickelbares lichtempfindliches Material nach Anspruch 5, worin B eine organische Base, gewählt aus der Gruppe, bestehend aus einem aromatischen oder aliphatischen Amin, einem aromatischen oder aliphatischen Diamin, einem Piperidin, einem Piperazin, einem Guanidin, einem cyclischen Guanidin, einem Amidin, einem cyclischen Amidin und einem Tetraalkylammoniumhydroxyd, bedeutet.

9. Wärmeentwickelbares lichtempfindliches Material nach Anspruch 1, worin M ein Atom, gewählt aus der Gruppe, bestehend aus Na, K, Cs und Ba, bedeutet.

10. Wärmeentwickelbares lichtempfindliches Material nach Anspruch 1, worin der Basenvorläufer in einer Menge von 0,001 bis 59 Gew.-%, bezogen auf das Gesamtgewicht der trockenen Überzugsschicht des lichtempfindlichen Materials, eingearbeitet wird.

11. Wärmeentwickelbares lichtempfindliches Material nach Anspruch 1, worin die Silberhalogenidemulsion einen Sensibilisierungsfarbstoff enthält.

12. Wärmeentwickelbares lichtempfindliches Material nach Anspruch 11, worin die Menge des Sensibilisierungsfarbstoffs 0,001 bis 20 g pro 100 g Silber der Silberhalogenidemulsion beträgt.

13. Wärmeentwickelbares lichtempfindliches Material nach Anspruch 1, worin das lichtempfindliche Material ein farbstofflieferndes Material enthält, das einen mobilen Farbstoff freisetzt oder liefert, entsprechend zu oder umgekehrt zu einer Reduktionsreaktion eines Silberhalogenids durch ein Reduktionsmittel als bildlieferndes Material.

14. Wärmeentwickelbares lichtempfindliches Material nach Anspruch 1, worin das lichtempfindliche Material wenigstens ein bildlieferndes Material, gewählt aus der Gruppe, bestehend aus einem Kuppler, einem Farbstoff, der positive Farbbilder durch ein photographisches Silberfarbstoffleichverfahren bilden kann, einem Farbstoff mit einer darin eingeführten stickstoffhaltigen heterocyclischen Ringgruppe, einem farbstoffliefernden Material, das einen mobilen Farbstoff durch eine Kupplungsreaktion mit einem Reduktionsmittel, das durch eine Oxidationsreduktionsreaktion mit einem Silberhalogenid oder einem organischen Silbersalz bei der Wärmeentwicklung oxidiert wird, freisetzt, einer nichtdiffundierbaren bildliefernden Verbindung, die einen Selbstringschluß in Gegenwart einer Base bewirkt, um einen diffundierbaren Farbstoff freizusetzen, jedoch den Farbstoff nicht freisetzt, wenn die Verbindung mit dem Oxidationsprodukt eines Entwicklungsmittels reagiert, und einer nicht diffundierbaren bildliefernden Verbindung, die selbst keinen Farbstoff freisetzt, jedoch einen Farbstoff freisetzt, wenn die Verbindung mit einem Reduktionsmittel reagiert, und einer verbundenen Donorakzeptorverbindung, die eine nicht diffundierbare bildliefernde Verbindung ist und eine Donorakzeptorreaktion in Gegenwart einer Base bewirkt, um einen diffundierbaren Farbstoff freizusetzen, jedoch im wesentlichen keinen Farbstoff freisetzt, wenn die Verbindung mit dem Oxidationsprodukt eines Entwicklungsmittels reagiert, enthält.

15. Wärmeentwickelbares lichtempfindliches Material nach Anspruch 14, worin das lichtempfindliche Material weiterhin ein farbstofflieferndes Material enthält, das einen mobilen Farbstoff als Ergebnis einer Oxidationsreduktionsreaktion mit Silberhalogenid oder einem organischen Silbersalz bei der Wärmeentwicklung freisetzt.

16. Wärmeentwickelbares lichtempfindliches Material nach Anspruch 1, worin das lichtempfindliche Material weiterhin wenigstens eine Schicht, gewählt aus einer Zwischenschicht, einer Filterschicht, einer Antilichthofschicht, einer Schutzschicht und einer Bildfixierschicht, vorgesehen auf dem Träger, umfaßt.

17. Wärmeentwickelbares lichtempfindliches Material nach Anspruch 1, worin der Basenvorläufer in die Silberhalogenidemulsionsschicht eingearbeitet ist.

18. Wärmeentwickelbares lichtempfindliches Material nach Anspruch 1, worin das lichtempfindliche Material weiterhin eine Bildfixierschicht, vorgesehen auf einem anderen Träger als dem für die lichtempfindliche Schicht, umfaßt und der Basenvorläufer in die Bildfixierschicht eingearbeitet ist.

19. Verfahren zur Herstellung eines Bildes, bei dem ein wärmeentwickelbares lichtempfindliches Material nach einem der Ansprüche 1 bis 18 wärmeentwickelt wird.

**Revendications**

1. Un matériau photosensible développable à chaud comprenant un support et une couche d'émulsion d'halogénure d'argent formée sur celui-ci, ledit matériau photosensible contenant comme précurseur de base au moins un composé choisi parmi les composés de formules générales (I) et (II):

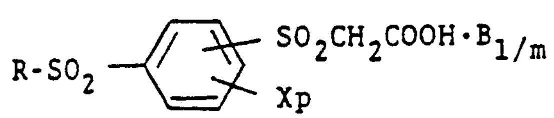
(I)

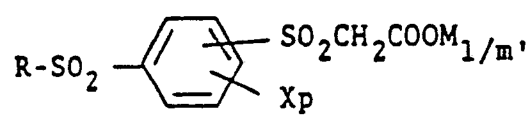
(II)

.dans lesquelles

R représente un groupe alkyle substitué ou non, un groupe cycloalkyle substitue ou non, un group alcényle substitué ou non, un groupe alcynyle substitué ou non, un groupe arylalkyle substitué ou non, un groupe aryle substitué ou non ou un groupe hétérocyclique, à la condition que R ne soit pas un groupe carboxyméthyle;

X représente un atome d'hydrogène ou d'halogène ou un groupe alkyle, un groupe cycloalkyle, un groupe arylalkyle, un groupe alcoxy, un groupe aryloxy, un groupe aryle, un groupe alkyl ou arylacylamino, un groupe alkyl ou arylacyle, un groupe cyano, un groupe alkylsulfonylamino, un groupe nitro, un groupe arylsulfonylamino, un groupe alkylsulfonyle, un groupe arylsulfonyle, un groupe sulfamoyle, un groupe sulfamoyle substitué, un groupe carbamoyle, un groupe carbamoyle substitué, un groupe alkylthio, un groupe arylthio, un groupe alcoxycarbonyle, un groupe aryloxycarbonyle, un groupe alkyl ou arylacyloxy et les groupes ci-dessus substitués sur le reste alkyle ou aryle, $—OM_{1/m'}$, $—COOM_{1/m'}$, $OH \cdot B_{1/m}$ (où —OH est phénolique) et $—COOH \cdot B_{1/m'}$;

p représente un entier de 0 à 4;

B représente une base monoacide ou une base diacide;

M représente un métal alcalin ou un métal alcalino-terreux; et

m est égal à 1 lorsque B représente une base monoacide et à 2 lorsque B représente une base diacide et m′ représente la valence de M.

2. Le matériau photosensible développable à chaud selon la revendication 1, caractérisé en ce que le noyau hétérocyclique représenté par R est un noyau hétérocyclique à 5 ou 6 chaînons contenant au moins 1 hétéroatome choisi parmi l'azote, l'oxygène et le soufre.

3. Le matériau photosensible développable à chaud selon la revendication 2, caractérisé en ce que ledit noyau hétérocyclique est choisi parmi un groupe pyridyle, un groupe pyrazolyle et un groupe imidazolyle.

4. Le matériau photosensible développable à chaud selon la revendication 1, caractérisé en ce que le substituant desdits groupes substitués représentés par R est choisi parmi un groupe alkyle, un groupe alkyle- ou arylsulfonyle, un groupe sulfamoyle, un groupe N-alkyl- ou N-arylsulfamoyle, un groupe carbamoyle, un groupe N-alkyl- ou N-arylcarbamoyle, un groupe alkyl ou arylsulfonamido, un groupe alkyl- ou arylacylamido, un atome d'halogène et un groupe hydroxycarbonyle.

5. Le matériau photosensible développable à chaud selon la revendication 1, caractérisé en ce que B est une base acide organique ayant un pKa de pas moins de 7 et ne contenant pas plus de 12 atomes de carbone.

6. Le matériau photosensible développable à chaud selon la revendication 5, caractérisé en ce que B a un point d'ébullition de pas moins de 150°C.

7. Le matériau photosensible développable à chaud selon la revendication 6, caractérisé en ce que B est une base azotée monoacide ou diacide exempte de soufre.

8. Le matériau photosensible développable à chaud selon la revendication 5, caractérisé en ce que B représente une base organique choisie parmi les amines aromatiques et aliphatiques, les diamines aromatiques ou aliphatiques, les pipéridines, les pipérazines, les guanidines, les guanidines cycliques, les amidines, les amidines cycliques et les hydroxydes de tétraalkylammonium.

9. Le matériau photosensible développable à chaud selon la revendication 1, caractérisé en ce que M représente un atome choisi parmi Na, K, Cs et Ba.

10. Le matériau photosensible développable à chaud selon la revendication 1, caractérisé en ce que ledit précurseur de base est incorporé en quantite de 0,001 à 50% en poids, par rapport au poids total de la couche appliquée sèche du matériau photosensible.

11. Le matériau photosensible développable à chaud selon la revendication 1, caractérisé en ce que ladite émulsion de l'halogènure d'argent contient un colorant sensibilisateur.

12. Le matériau photosensible développable à chaud selon la revendication 11, caractérisé en ce que la quantité du colorant sensibilisateur est de 0,001 à 20 g par 100 g d'argent de l'émulsion d'halogénure d'argent.

13. Le matériau photosensible développable à chaud selon la revendication 1, caractérisé en ce qu'il contient un produit générateur de colorant, qui libère ou produit un colorant mobile correspondant directement ou inversement à une réaction de réduction d'un halogénure d'argent par un agent réducteur, comme matière formant l'image.

14. Le matériau photosensible développable à chaud selon la revendication 1, caractérisé en ce qu'il contient au moins un composé formateur d'image choisi parmi un coupleur; un colorant qui est capable de former des images en couleur positives par un procédé photographique de blanchiment à l'argent des colorants; un colorant dans lequel est introduit un groupe hétérocyclique azoté; un composé formateur de colorant qui libère un colorant mobile par une réaction de couplage avec un agent réducteur qui est oxydé par une réaction d'oxydo-réduction avec un halogénure d'argent ou un sel organique d'argent par développement à chaud; un composé non diffusible formateur d'image qui provoque une auto-cyclisation en présence d'une base en libérant un colorant diffusible, mais ne libère pas le colorant lorsque le composé réagit avec produit d'oxydation d'un agent développateur, et un composé non diffusible formateur d'image qui ne libère pas un colorant par lui-même mais libère un colorant lorsque le composé réagit avec un agent réducteur, et une composé donneur-accepteur lié qui est un composé non diffusible formateur d'image et provoque une réaction donneur-accepteur en présence d'une base en libérant un colorant diffusible, mais ne libère pratiquement pas le colorant lorsque le composé réagit avec le produit d'oxydation d'un agent développateur.

15. Le matériau photosensible développable à chaud selon la revendication 14, caractérisé en ce qu'il contient en outre un composé formateur de colorant qui libère un colorant mobile par suite d'une réaction d'oxydo-réduction avec un halogénure d'argent ou un sel organique d'argent par développement à chaud.

16. Le matériau photosensible développable à chaud selon la revendication 1, caractérisé en ce qu'il comprend en outre au moins sur le support une couche supplémentaire choisie parmi une couche intermédiaire, une couche filtrante, une couche antihalo, une couche protectrice et une couche fixatrice d'image.

17. Le matériau photosensible développable à chaud selon la revendication 1, caractérisé en ce que ledit précurseur de base est incorporé dans la couche d'émulsion d'halogénure d'argent.

18. Le matériau photosensible développable à chaud selon la revendication 1, caractérisé en ce qu'il comprend en outre une couche fixatrice d'image sur un support autre que celui de la couche photosensible et en ce que ledit précurseur de base est incorporé dans la couche fixatrice d'image.

19. Un procédé pour produire une image qui consiste à développer à chaud un matériau photosensible développable à chaud selon l'une quelconque des revendications 1 à 18.